# NEW EUROPEAN PATENT SPECIFICATION

(11) **EP 0 608 447 B2**
(45) Date of publication and mention of the opposition decision: **14.03.2007**
(45) Mention of the grant of the patent: 17.10.2001
(21) Application number: 93101111.8
(22) Date of filing: 26.01.1993
(51) Int. Cl.: C08F 10/00, C07C 2/32, B01J 31/18, B01J 31/12

(54) **Process for the preparation of a catalyst for olefin polymerization**
Verfahren zur Herstellung eines Olefinpolymerisationskatalysator
Procédé de préparation d'un catalyseur pour polymérisation d'oléfines

(43) Date of publication of application: 03.08.1994
(73) Proprietor: ConocoPhillips Company, Bartlesville, OK 74004 (US)
(72) Inventor: Reagan, William Kevin, Stillwater, Minnesota 55082 (US); Freeman, Jeffrey Willis, Bartlesville, Oklahoma 74006 (US); Conroy, Brian Keith, Batavia, Illinois 60510 (US); Pettijohn, Ted Matthew, Bartlesville, Oklahoma 74006 (US); Benham, Elizabeth Ann, Bartlesville, Oklahoma 74006 (US)
(74) Representative: Dost, Wolfgang

(56) References cited:
- EP-A- 0 416 304
- EP-A1- 0 548 805
- GB-A1- 2 271 116
- NL-A1- 9 301 583

## Description

This invention relates to chromium catalysts or cocatalysts to trimerize, oligomerize and/or polymerize olefins. This invention also relates to a process to trimerize, oligomerize and/or polymerize olefins.

Supported chromium oxide catalysts have been a dominant factor in the production of olefin polymers, such as polyethylene or copolymers of ethylene and hexene. These catalysts can be used in a variety of polymerization processes. However, most known chromium compounds must be supported to be catalytically active. Furthermore, most supported chromium compounds are useful only for olefin polymerization. If an olefin copolymer is desired, the polymerization process becomes more complex in that two different monomers must be fed to the polymerization reactor.

Olefin trimerization and oligomerization catalysts are also known in the art, but usually lack selectivity to a desired product and also have a low product yield. However, olefin trimerization and/or oligomerization, if done efficiently, is a process to provide useful olefins. These olefinic products can be further trimerized, oligomerized and/or, optionally, incorporated into a polymerization process.

In EP-A-416 304 there is described a chromium-containing compound having the formula:

Cr₅(C₄H₄N)₁₀(C₄H₃O)₄; [Cr(C₄H₄N)₄][Na]₂·2(OC₄H₃);

[Cr(C₄H₄N)₅(OC₄H₃)][Na]₂·4(OC₄H₃);

or

Cr(NC₄H₄)₃Cl(O₂C₂H₄(CH₃)₂)₃Na.

In accordance with an aspect of the above application, such chromium-containing compounds are prepared from a reaction mixture comprising a chromium salt, a metal amide, and any electron pair donor solvent, such as an ether. The catalyst systems can be used, either supported or unsupported, to trimerize and/or polymerize olefins.

The chromium salt can be one or more organic or inorganic chromium salts, wherein the chromium oxidation state is from 0 to 6. As used in this disclosure, chromium metal is included in this definition of a chromium salt. Generally, the chromium salt will have a formula of CrXₙ, wherein X can be the same or different and can be any organic or inorganic radical, and n is an integer from 1 to 6. Exemplary organic radicals can have from about 1 to about 20 carbon atoms per radical, and are selected from alkyl, alkoxy, ester, ketone, and/or amido radicals. The organic radicals can be straight-chained or branched, cyclic or acyclic, aromatic or aliphatic, and can be made of mixed aliphatic, aromatic, and/or cycloaliphatic groups. Exemplary inorganic radicals include, but are not limited to halides, sulfates, and/or oxides.

Preferably, the chromium salt of EP-A-416 304 is a halide, such as chromous chloride, chromic chloride, chromous bromide, chromic bromide, chromous iodide, chromic iodide, and mixtures thereof. Most preferably, the chromium salt is a chloride, such as chromous chloride and/or chromic chloride, due to simple separation of the reaction by-products such as sodium chloride, as well as relatively low cost.

The metal amide of EP-A-416 304 can be any metal amide that will react with a chromium salt to form a chromium-amido complex. Broadly, the metal amide can be any heteroleptic or homoleptic metal complex or salt, wherein the amide radical can be any nitrogen-containing organic radical. The metal amide can be either affirmatively added to the reaction, or generated in-situ. Generally, the metal amide will have from about 1 to about 20 carbon atoms.

Exemplary preferred metal amides include, but are not limited to, lithium dimethylamide, lithium diethylamide, lithium diisopropylamide, lithium dicyclohexylamide, sodium bis(trimethylsilyl)amide, sodium indolide, alkali metal and alkaline earth metal pyrrolides, and mixtures of two or more thereof. Most preferred are the above metal pyrrolides such as lithium pyrrolide, sodium pyrrolide, potassium pyrrolide, and cesium pyrrolide, because of high reactivity and activity with the other reactants. Examples of substituted pyrrolides include, but are not limited to sodium 2,5-dimethyl pyrrolide and/or 3,4-dimethyl pyrrolide. When the metal amide is a pyrrolide ligand, the resultant chromium compound is a chromium pyrrolide.

The ether in the reaction mixture of EP-A-416 304 can be one or more ether compounds to effect a reaction between the chromium salt and the metal amide. While not wishing to be bound by theory, it is believed that the ether can be a reaction solvent, as well as a possible reactant. The ether can be any aliphatic and/or aromatic compound containing an R-O-R functionality, wherein the R groups can be the same or different, but preferably is not hydrogen. Preferred ethers are aliphatic ethers, for safety reasons in that aromatic ethers are human toxins. Furthermore, the preferred ethers are those which facilitate a reaction between a chromium halide and a Group IA or Group IIA metal pyrrolide, and also can be easily removed from the reaction mixture. Exemplary compounds include, but are not limited to, tetrahydrofuran, dioxane, diethylether, dimethoxyethane (glyme), diglyme, triglyme, and mixtures of two or more thereof. Most preferably, the ether is selected from tetrahydrofuran, derivatives of tetrahydrofuran, dimethoxyethane, derivatives of dimethoxyethane, and mixtures thereof, for the reasons given above, as well as the reason that the preferred salt of an amine is soluble in these ethers.

The three reactants of EP-A-416 304 can be combined in any manner under conditions suitable to form a solution comprising one or more of the chromium compounds. The reaction preferably occurs in the absence of oxygen and moisture and therefore under an inert atmosphere, such as nitrogen and/or argon. The reaction pressure can be any pressure sufficient to maintain the reactants in a liquid state. Generally, pressure within the range of from about atmospheric pressure to about three atmospheres are acceptable. For ease of operation atmospheric pressure is generally employed.

The precipitated chromium compounds can be recovered by any method known in the art. The simplest procedure to remove the precipitated chromium compounds is by filtration.

In accordance with one aspect of the present invention, a catalyst system can be prepared in accordance to the process of claim 1. The metal source is a chromium compound, the metal alkyl is an aluminum, a lithium, a magnesium, or a zinc alkyl. The optional unsaturated hydrocarbon, for example toluene, can serve as the mutual solvent. In the absence of such an unsaturated hydrocarbon serving as the mutual solvent, there can be used cyclohexane. These catalyst systems can further comprise a catalyst support.

The metal source, similar to the earlier discussed chromium salt, is one or more organic or inorganic chromium compounds, wherein the chromium oxidation state is from 0 to 6. As used in this disclosure, chromium metal is included in this definition of a chromium compound. Generally, the metal source will have a formula of CrXₙ, wherein X can be the same or different and can be any organic or inorganic radical, and n is an integer from 0 to 6. Exemplary organic radicals can have from about 1 to about 20 carbon atoms per radical, and are selected from alkyl, alkoxy, ester, ketone, and/or amino radicals. The organic radicals can be straight-chained or branched, cyclic or acyclic, aromatic or aliphatic, and/or cycloaliphatic groups. Exemplary inorganic radicals include, but are not limited to halides, sulfates, and/or oxides.

Preferably, the metal source is a chromium(II)- and/or chromium(III)-containing compound which can yield a catalyst system with improved trimerization activity. Most preferably, the metal source is a chromium(III) compound because of ease of use, availability, and enhanced catalyst system activity. Exemplary chromium(III) compounds include, but are not limited to, chromium carboxylates, chromium naphthenates, chromium halides, and/or chromium dionates. Specific exemplary chromium(III) compounds include, but are not limited to, chromium(III) 2,2,6,6-tetramethylheptanedionate [Cr(TMHD)₃], chromium(III)2-ethylhexanoate [Cr(DH)₃], chromium(III)naphthenate [Cr(Np)₃], chromium(III) chloride, chromium (III) tris(2-ethylhexanoate), chromic bromide, chromic chloride, chromic fluoride, chromium (III) oxy-2-ethylhexanoate, chromium (III) dichloroethylhexanoate, chromium (III) acetylacetonate, chromium (III) acetate, chromium (III) butyrate, chromium (III) neopentanoate, chromium (III) laurate, chromium (III) stearate, and/or chromium (III) oxalate.

Specific exemplary chromium (II) compounds include, but are not limited to, chromous fluoride, chromous chloride, chromous bromide, chromous iodide, chromium (II) bis(2-ethylhexanoate), chromium (II) acetate, chromium (II) butyrate, chromium (II) neopentanoate, chromium (II) laurate, chromium (II) stearate, and/or chromium (II) oxalate.

The pyrrole-containing compound can be any pyrrole-containing compound that will react with a chromium salt to form a chromium pyrrolide complex. As used in this disclosure, the term "pyrrole-containing compound" refers to hydrogen pyrrolide, i.e., pyrrole, (C₄H₅N), derivatives of hydrogen pyrrolide, as well as metal pyrrolide complexes. A "pyrrolide" (or a "pyrrole" as referred to in the first aspect of the invention) can be any compound comprising a 5-membered, nitrogen-containing heterocycle, such as pyrrole, derivatives of pyrrole, and mixtures thereof. Broadly, the pyrrole-containing compound can be pyrrole and/or any heteroleptic or homoleptic metal complex or salt, containing a pyrrolide radical, or ligand. The pyrrole-containing compound can be either affirmatively added to the reaction, or generated in-situ.

Generally, the pyrrole-containing compound will have from about 4 to about 20 carbon atoms per molecule. Exemplary pyrrolides (or pyrroles) include hydrogen pyrrolide (pyrrole), derivatives of pyrrole, substituted pyrrolides (or pyrroles), lithium pyrrolide, sodium pyrrolide, potassium pyrrolide, cesium pyrrolide, and/or the salts of substituted pyrrolides, because of high reactivity and activity with the other reactants. Examples of substituted pyrrolides (or pyrroles) include, but are not limited to, pyrrole-2-carboxylic acid, 2-acetylpyrrole, pyrrole-2-carboxaldehyde, tetrahydroindole, 2,5-dimethylpyrrole, 2,4-dimethyl-3-ethylpyrrole, 3-acetyl-2,4-dimethylpyrrole, ethyl-2,4-dimethyl-5-(ethoxycarbonyl)-3-pyrrole-propionate, ethyl-3,5-dimethyl-2-pyrrole-carboxylate.

In accordance with the present invention pyrrole itself (hydrogen pyrrolide) or a substituted pyrrole is recognized as a suitable pyrrolide. Pyrrole is a preferred hydrogen pyrrolide. Substituted pyrroles are illustrated below.

The most preferred pyrrole-containing compounds used in a trimerization catalyst system are selected from hydrogen pyrrolide, i.e., pyrrole (C₄H₅N) and/or 2,5-dimethyl pyrrole. While all pyrrole-containing compounds can produce catalysts with high activity and productivity, use of pyrrole and/or 2,5-dimethylpyrrole can produce a catalyst system with enhanced activity and selectivity to a desired trimerized product, such as, for example, the trimerization of ethylene to 1-hexene, as well as decreased polymer production.

The metal alkyl, also referred to earlier as an activating compound, is a heteroleptic or homoleptic metal alkyl compound of any of the following metals: Al, Li, Mg, and Zn. One or more metal alkyls can be used. The ligand(s) on the metal can be aliphatic and/or aromatic. Preferably, the ligand(s) are any saturated or unsaturated aliphatic radical. The metal alkyl can have any number of carbon atoms. However, due to commercial availability and ease of use, the metal alkyl will usually comprise less than about 70 carbon atoms per metal alkyl molecule and preferably less than about 20 carbon atoms per molecule. Preferred metal alkyls thus include alkylaluminum compounds, alklymagnesium compounds, alkylzinc compounds and/or alkyllithium compounds. Exemplary metal alkyls include, but are not limited to, n-butyllithium, s-butyllithium, t-butyllithium, diethylmagnesium, diethylzinc, triethylaluminum, trimethylaluminum, triisobutylaluminum, and mixtures thereof.

Most preferably, activating compounds are selected from non-hydrolyzed, i.e., not pre-contacted with water, alkylaluminum compounds, derivatives of alkylaluminum compounds, halogenated alkylaluminum compounds, and mixtures thereof for improved product selectivity, as well as improved catalyst system reactivity, activity, and/or productivity. Exemplary compounds include, but are not limited to, triethylaluminum, tripropylaluminum, tributylaluminum, diethylaluminum chloride, diethylaluminum bromide, diethylaluminum ethoxide, diethylaluminum sesquichloride, and mixtures thereof for best catalyst system activity and product selectivity. The most preferred alkylaluminum compound is triethylaluminum, for best results in catalyst system activity and product selectivity, as well as commercial availability.

When a trimerization catalyst system is the desired product, the activating compound must be at least one non-hydrolyzed alkylaluminum compound, expressed by the general formulae AlR₃, AlR₂X, AlRX₂, AlR₂OR, AIRXOR, and/or Al₂R₃X₃, wherein R is an alkyl group and X is a halogen atom. Exemplary compounds include, but are not limited to, triethylaluminum, tripropylaluminum, tributylaluminum, diethylaluminumchloride, diethylaluminumbromide, diethylaluminumethoxide, diethylaluminum phenoxide, ethylaluminumethoxychloride, and/or ethylaluminum sesquichloride. Preferably, the activating compound for a trimerization catalyst system is a trialkylaluminum compound, AlR₃, for reasons given above. The most preferred trialkylaluminum compound is triethylaluminum, for reasons given above.

The chromium catalyst system prepared by the above process of the present invention can be used, either as a supported and/or unsupported catalyst system, for olefin trimerization, oligomerization and/or polymerization. A supported chromium catalyst system can be prepared with any support useful to support a chromium catalyst. Exemplary catalyst supports include, but are not limited to, zeolites, inorganic oxides, either alone or in combination, phosphated inorganic oxides, and mixtures thereof. Particularly preferred are supports selected from silica, silica-alumina, alumina, fluorided alumina, silated alumina, thoria, aluminophosphate, aluminum phosphate, phosphated silica, phosphated alumina, silica-titania, coprecipitated silica/titania, fluorided/silated alumina, and mixtures, thereof, being presently preferred, as well as any one or more of these supports which can contain chromium. The presently most preferred catalyst support, because of the greatest trimerization activity, is aluminophosphate, as disclosed in U.S. Patent 4,364,855 (1982).

The amount of chromium pyrrolide compound per gram of support can be expressed in different, yet equivalent terms, such as, for example, moles of chromium per gram of support. Usually, less than about 8.6 x 10⁻³ moles of chromium per gram of support is sufficient. Preferably, about 1.7 x 10^{- 6} to about 1.7 x 10⁻⁵ to 8.6 x 10⁻⁴ moles of chromium per gram of support are used, for reasons given above.

After the support is added and thoroughly combined with the chromium pyrrolide, it can be collected by filtration, vacuum dried, then a metal alkyl as an activating compound, usually as a solution of one or more Lewis acids and/or metal alkyls, preferably in hydrocarbon compound solvent, is added to the support/chromium pyrrolide mixture. An active, supported catalyst system then can be collected by filtration. As used in this disclosure, a Lewis acid is defined as any compound that is an electron acceptor.

Preferably, the activating compound, i.e., the metal alkyl, is a compound that can be considered both a Lewis acid and a metal alkyl. As more broadly described in the present application, the activating compound can have any number of carbon atoms. However, due to commercial availability and ease of use, the activating compound will usually comprise less than about 70 carbon atoms per metal alkyl molecule and preferably less than about 20 carbon atoms per molecule. Activating compounds which are both a metal alkyl and a Lewis acid include alkylaluminum compounds, alkylmagnesium, alkylzinc, and/or alkyllithium compounds. Exemplary metal alkyls include, but are not limited to, n-butyllithium, s-butyllithium, t-butyllithium, diethylmagnesium, dibutylmagnesium, diethylzinc, triethylaluminum, trimethylaluminum, triisobutylaluminum, and mixtures thereof. Most preferably, activating compounds are selected from non-hydrolyzed, i.e., not pre-contacted with water, alkylaluminum compounds, derivatives of alkylaluminum compounds, halogenated alkylaluminum compounds, and mixtures thereof for improved product selectivity, as well as improved catalyst system reactivity, activity, and/or productivity. Exemplary compounds include, but are not limited to, triethylaluminum, tripropylaluminum, tributylaluminum, diethylaluminum chloride, diethylaluminum bromide, diethylaluminum ethoxide, ethylaluminum sesquichloride, and mixtures thereof for best catalyst system activity and product selectivity. The most preferred alkylaluminum compound is triethylaluminum, for best results, in catalyst system activity and product selectivity.

Any amount of metal alkyl is sufficient to activate and/or react with the chromium pyrrolide catalyst. Usually about 200 g of metal alkyl per gram of chromium can be used. Preferably, about 1 to about 100 g of metal alkyl per gram of chromium pyrrolide, and most preferably about 5 to about 30 g of a metal alkyl per gram of chromium pyrrolide are used, for best catalyst activity. However, the amount of metal alkyl employed can vary with the catalyst support used. For example, if the support is silica and/or alumina, too much of the metal alkyl can decrease catalyst activity. However, a similar amount of metal alkyl used with an aluminophosphate support does not always significantly decrease catalyst activity.

Formation of stable and active catalyst systems can take place in the presence of an unsaturated hydrocarbon. As discussed hereinbelow an unsaturated hydrocarbon can be present either during the initial contacting of the metal source, the pyrrole-containing compound, and the metal alkyl, or can be introduced directly into a trimerization, oligomerization and/or polymerization reactor. Furthermore, one or more of the olefin reactants can be considered the unsaturated hydrocarbon.

As more broadly described herein, the hydrocarbon compound used as a solvent can be any combination of one or more aromatic or aliphatic unsaturated hydrocarbon compounds. While not wishing to be bound by theory, it is believed that an unsaturated hydrocarbon compound acts as more than a solvent, and can be a reactant and/or a stabilizing component during and/or subsequent to formation of an inventive catalyst system. Exemplary unsaturated hydrocarbon compounds, such as a solvent, can be any unsaturated hydrocarbon compound that can dissolve the metal alkyl. In accordance, a further aspect of the present invention in addition to the aromatic compounds having from about 6 to about 50 carbon atoms per molecule as a solvent as described in our above application there can be used any unsaturated aliphatic hydrocarbon comprising less than about 20 carbon atoms per molecule. Specific exemplary unsaturated aliphatic compounds include 1-hexene, 1,3-butadiene, and mixtures thereof. Ethylene can be a trimerization and/or oligomerization reactant. Specific exemplary unsaturated aromatic hydrocarbon compounds include, but are not limited to, toluene, benzene, xylene, mesitylene, hexamethylbenzene, and mixtures thereof. As described in our above application, the most preferred unsaturated aromatic hydrocarbon compound solvent is toluene, for ease of removal and minimal interference with the resultant catalyst system.

The unsaturated hydrocarbon compound can be present either during the initial contacting of a chromium pyrrolide and metal alkyl i.e., prior to introduction into a trimerization, oligomerization and/or polymerization reactor, or the unsaturated hydrocarbon compound can be introduced directly into the reactor. As stated above, one or more of the olefin reactants can be considered the unsaturated hydrocarbon. Preferably, the unsaturated hydrocarbon is present during the initial contacting of a chromium pyrrolide and metal alkyl in order to stabilize the resultant catalyst system. In the absence of an unsaturated hydrocarbon, the resultant catalyst system can deactivate and lose activity over a period of time.

While any amount of unsaturated hydrocarbon compound can be used, too much or too little can adversely affect catalyst system activity. Therefore, preferably, the resultant catalyst system is stripped of any excess unsaturated aromatic hydrocarbon. Stripping of excess unsaturated aromatic hydrocarbon can be accomplished by any method known in the art, such as, for example, solvent removal methods. Exemplary removal methods include, but are not limited to, filtration, vacuum drying, drying under an inert atmosphere, and combinations thereof. While not wishing to be bound by theory, it is believed that the remaining unsaturated hydrocarbon can stabilize the resultant catalyst system. If no unsaturated hydrocarbon is present, it is believed that the catalyst system can lose activity.

Any aromatic or aliphatic unsaturated hydrocarbon can be used. Preferably, an unsaturated hydrocarbon initially is present in the reaction mixture and most preferably, an aromatic hydrocarbon and/or ethylene initially is present to produce a highly active catalyst in terms of activity and selectivity, as well as a stable catalyst system. The unsaturated hydrocarbon can have any number of carbon atoms per molecule. Usually, the unsaturated hydrocarbon will comprise less than about 70 carbon atoms per molecule, preferably less than about 20 carbon atoms per molecule, due to commercial availability and ease of use.

The unsaturated hydrocarbon can be a gas, liquid, or solid. Preferably, to effect thorough contacting and mixing of the chromium salt, pyrrole-containing compound, and metal alkyl, the unsaturated hydrocarbon will be in a liquid and/or dissolved state. Exemplary unsaturated aliphatic hydrocarbons include, but are not limited to, ethylene, 1-hexene, 1,3-butadiene, and mixtures thereof. The most preferred unsaturated aliphatic hydrocarbon is ethylene, since ethylene can be a reactant during trimerization, oligomerization, and/or polymerization. Exemplary unsaturated aromatic hydrocarbons include, but are not limited to, toluene, benzene, xylene, mesitylene, hexamethylbenzene, and mixtures thereof. Unsaturated hydrocarbons are preferred in order to improve catalyst system stability, as well as improve catalyst system activity. The most preferred unsaturated aromatic hydrocarbon is toluene, for best resultant catalyst system stability and activity.

If an unsaturated aromatic hydrocarbon is added prior to introduction of the chromium compound(s) to a trimerization, oligomerization and/or polymerization reactor, removal of, or stripping, the unsaturated aromatic hydrocarbon prior to introduction of the chromium compound(s) into a reactor can improve catalyst system activity and/or product selectivity. Removal of the unsaturated aromatic hydrocarbon can be done in any manner known in the art, such as, for example, flashing or evaporation. The resultant product is a concentrated, or saturated, solution of an inventive catalyst system.

When the unsaturated aromatic hydrocarbon is removed prior to introduction to a reactor, the concentrated, or saturated, solution of an inventive catalyst system can be dissolved in a solvent compatible with the trimerization, oligomerization and/or polymerization process to improve ease of handling the inventive catalyst system. Generally, the solvent is the same as the reactor diluent. The solvents include cyclohexane, isobutane, hexane, pentane, mixtures thereof, and unsaturated hydrocarbons.

The reaction, optionally, also can take place in the presence of a halide source. The presence of a halide source in the reaction mixture can increase catalyst system activity and productivity, as well as increase product selectivity. Exemplary halides include, but are not limited to, fluoride, chloride, bromide, and/or iodide. Due to ease of use and availability, chloride is the preferred halide. Based on improved activity, productivity, and/or selectivity, bromide is the most preferred halide.

The halide source can be any compound containing a halogen. Exemplary compounds include, but are not limited to, compounds with a general formula of RₘXₙ, wherein R can be any organic and/or inorganic radical, X can be a halide, selected from fluoride, chloride, bromide, and/or iodide, and m and n each are numbers greater than 0. If R is an organic radical, preferably R has from about 1 to about 70 carbon atoms per radical, most preferably from 1 to 20 carbon atoms per radical, for best compatibility and catalyst system activity. If R is an inorganic radical, preferably R is selected from aluminum, silicon, germanium, hydrogen, boron, lithium, tin, gallium, indium, lead, and mixtures thereof. Specific exemplary compounds include, but are not limited to, methylene chloride, chloroform, benzylchloride, silicon tetrachloride, tin (II) chloride, tin (IV) chloride, germanium tetrachloride, boron trichloride, aluminum tribromide, aluminum trichloride, 1,4-di-bromobutane, and/or 1-bromobutane.

Furthermore, the chromium source, the metal alkyl, and/or unsaturated hydrocarbon can contain and provide a halide to the reaction mixture. Preferably, the halide source is an alkylaluminum halide and is used in conjunction with alkylaluminum compounds due to ease of use and compatibility, as well as improved catalyst system activity and product selectivity. Exemplary alkylaluminum halides include, but are not limited to, diisobutylaluminum chloride, diethylaluminum chloride, ethylaluminum sesquichloride, ethylaluminum dichloride, diethylaluminum bromide, diethylaluminum iodide, and mixtures thereof.

When a trimerization catalyst system is the desired product, preferably, the reaction mixture comprises a halide source. Furthermore, most preferably, the halide source is selected from tin (IV) halides, germanium halides, and mixtures thereof. The halide source, most preferably, is combined with the chromium source and pyrrole-containing compound prior to addition of the metal alkyl, i.e., the chromium source and pyrrole-containing compound are pre-treated with a halide source, to increase catalyst system productivity.

The amount of each reactant used to prepare a trimerization catalyst system can be any amount sufficient that, when combined with one or more olefins, trimerization, as defined in this disclosure, occurs. Usually, to prepare a trimerization catalyst system, about one mole of chromium, as the element chromium (Cr), can be combined with about 1 to about 50 moles of pyrrole-containing compound and about 1 to about 75 moles of aluminum, as the element, in an excess of unsaturated hydrocarbon. If an optional halide source is present, usually about 1 to about 75 moles of halide, as the element, are present. Preferably, about 1 mole of chromium, calculated as the element chromium (Cr), can be combined with about 1 to about 15 moles of pyrrole-containing compound and about 5 to about 40 moles of aluminum, calculated as the element aluminum (Al), in an excess of unsaturated hydrocarbon. If an optional halide source is present, preferably about 1 to about 30 moles of halide, calculated as elemental halide (X), are present. In general terms, the latter mole ratios are preferred according to the process of the present invention, wherein about 5 to about 40 moles of metal alkyl (not necessarily an aluminum alkyl) are used. Most preferably, about one mole of chromium, as the element (Cr), is combined with two to four moles of pyrrole-containing compound and 10 to 20 moles of aluminum, as the element (Al), in an excess of unsaturated hydrocarbon. If an optional halide source is present, most preferably 2 to 15 moles of halide, as an element (X), are present.

An excess of pyrrole-containing compound does not appear to improve catalyst system activity, productivity, and/or selectivity. An unsaturated hydrocarbon can improve catalyst system stability, activity, and/or selectivity. An excess of the unsaturated hydrocarbon can harm catalyst system selectivity and/or activity. Too much alkylaluminum can decrease catalyst system activity and product selectivity. Too little alkylaluminum can result in incomplete formation of a catalyst system, which in turn, can result in low catalyst system activity and increased formation of undesired polymeric by-products. An excess of an optional halide source can deactivate a catalyst system, and therefore can result in decreased catalyst system activity. As stated earlier, presence of a halide source can increase catalyst system activity and product selectivity.

In accordance with a further aspect of the invention preferably the pyrrole-containing compound is present in the reaction mixture together with the metal source prior to the introduction of the metal alkyl. If this order of addition is followed, a better catalyst system, in terms of product selectivity and catalyst system activity and productivity, can be produced.

The reaction preferably occurs in the absence of oxygen, which can deactivate the catalyst, and under anhydrous conditions, i.e., in the initial absence of water. Therefore a dry, inert atmosphere, such as nitrogen and/or argon is most preferred. Additionally, the metal alkyl is a non-hydrolyzed metal alkyl.

The reaction pressure can be any pressure which does not adversely affect the reaction. Generally, pressures within the range of from about atmospheric pressure to about three atmospheres are acceptable. For ease of operation atmospheric pressure is generally employed.

The reaction temperature can be any temperature. In order to effectuate a more efficient reaction, temperatures which maintain the reaction mixture in a liquid state, for the reasons given above, are preferred.

The reaction time can be any amount of time necessary for the reaction to occur. The reaction can be considered a dissolution process; any amount of time which can dissolve substantially all reactants is sufficient. Depending on the reactants, as well as the reaction temperature and pressure, reaction time can vary. Usually, times of less than about 1 day can be sufficient. Usually, reaction time is less than about 60 minutes. Under optimum conditions, the reaction time can be within the range of from about 1 second to about 15 minutes. Longer times usually provide no additional benefit, and shorter times may not allow sufficient time for complete reaction.

A heterogeneous, i.e., supported, catalyst system can be prepared in accordance with still a further aspect of the invention in-situ in the reactor by adding solid support directly to the reactor. As stated earlier, exemplary catalyst supports include, but are not limited to, zeolites, inorganic oxides, either alone or in combination, phosphated inorganic oxides, and mixtures thereof. Particularly preferred are supports selected from silica, silica-alumina, alumina, fluorided alumina, silated alumina, thoria, aluminophosphate, aluminum phosphate, phosphated silica, phosphated alumina, silica-titania, coprecipitated silica/titania, fluorided/silated alumina, and mixtures, thereof, being presently preferred, as well as any one or more of these supports which can contain chromium. The presently most preferred catalyst support, because of the greatest trimerization activity, is aluminophosphate, as disclosed in U.S. Patent 4,364,855. In-situ preparation of a heterogeneous catalyst system, used in a trimerization or oligomerization process, can decrease undesirable formation of polymer.

Heterogeneous trimerization, oligomerization, and/or polymerization catalyst systems can also be prepared in accordance with this aspect of the invention by forming a reaction mixture comprising the metal source, a pyrrole-containing compound, a metal alkyl, an unsaturated hydrocarbon, and an inorganic oxide, as disclosed earlier. Optionally, as disclosed earlier, a halide source can be added. Reaction stoichiometries and reaction conditions are the same as those disclosed herein above.

Any excess of chromium source, in relation to the inorganic oxide catalyst support, is sufficient. However, usually, less than about 5 g of chromium pyrrolide compound per gram of catalyst support is sufficient. Preferably, about 0.001 to about 0.01 to 0.5 g of chromium pyrrolide compound, or metal source, per gram of support is used for best support loading and most efficient use of the reagents. The amount of chromium pyrrolide, or metal source, per gram of support can be expressed in different, yet equivalent terms, such as moles of chromium per gram of support. Usually, less than about 8.6 x 10⁻³ moles of chromium per gram of support is sufficient. Preferably, about 1.7 x 10⁻⁶ to about 1.7 x 10⁻⁵ to 8.6 x 10⁻⁴ moles of chromium per gram of support are used, for reasons given above.

The resultant heterogeneous catalyst system can be collected by filtration, to recover a solid catalyst system product. The solid catalyst system is preferably kept under a dry, inert atmosphere to maintain chemical stability and reactivity. EP-A-417 477 describes polymerization catalyst and cocatalyst systems, the cocatalyst including the chromium compounds of EP-A-416 304. Generally, polymerization catalyst systems are considered either chromium catalysts (also known as "Phillips Catalysts") or titanium, zirconium and/or vanadium-containing catalysts.

Any chromium catalyst system known in the art can be used. Commercially available chromium catalyst systems typically comprise chromium, at least a portion of which is in the hexavalent state, supported on an inorganic oxide; optionally, the polymerization catalyst system can further comprise a metal alkyl cocatalyst. Exemplary chromium catalyst systems include, but are not limited to, those disclosed in U.S. Patents 3,887,494; 3,900,457; 4,053,436; 4,151,122; 4,294,724; 4,392,990; and 4,405,501.

Any titanium, zirconium and/or vanadium-containing catalyst system known in the art can also be used. Commercially available titanium, zirconium and/or vanadium catalyst system typically comprise complexes of transition metal halides with organometallic compounds. Exemplary magnesium/titanium catalysts include, but are not limited to, those disclosed in U.S. Patents 4,394,291; 4,326,988; and 4,347,158.

The amount of novel trimerization and/or oligomerization cocatalyst systems, including the inventive chromium compounds used as a cocatalyst can be any amount sufficient to generate a comonomer that can be incorporated into the polymer product. The chromium catalyst systems prepared by the various aspects of the present invention can serve as cocatalysts in conjunction with the titanium, zirconium and/or vanadium-containing catalysts discussed above.

The process of the above aspect of the invention in combining the metal source, and pyrrole-containing compound, and the metal alkyl, and, preferably an unsaturated hydrocarbon, avoids the need to isolate the first reaction product as per the processes described in our above-mentioned applications. Furthermore, the catalyst systems produced by the process of this aspect of the invention have improved productivity and selectivity to the desired trimerization product, for example in producing one-hexene from ethylene.

### Polymerization Reactants

Reactants applicable for use in polymerization with the catalyst systems and cocatalyst systems and processes of this invention are olefinic compounds which can polymerize, i.e., react the same or with other olefinic compounds. Catalyst systems of the invention can be used to polymerize at least one linear or branched mono-1-olefin having about 2 to about 8 carbon atoms. Exemplary compounds include, but are not limited to, ethylene, propylene, 1-butene, 1-pentene, 1-hexene, 1-octene, and mixtures thereof.

The catalyst systems of the present invention are also useful in tri-, poly- or oligomerization processes using olefin compounds having from about 2 to about 30 carbon atoms per molecule and having at least one olefinic double bond. Exemplary mono-olefin compounds include, but are not limited to, acyclic and cyclic olefins such as ethylene, propylene, 1-butene, 2-butene, isobutylene, 1-pentene, 2-pentene, 1-hexene, 2-hexene, 3-hexene, 1-heptene, 2-heptene, 3-heptene, the four normal octenes, the four normal nonenes, and mixtures of any two or more thereof. Exemplary diolefin compounds include, but are not limited to, 1,3-butadiene, isoprene, 1,4-pentadiene, and 1,5-hexadiene. If branched and/or cyclic olefins are used as reactants, while not wishing to be bound by theory, it is believed that steric hindrance could hinder the trimerization process. Therefore, the branched and/or cyclic portion(s) of the olefin preferably should be distant from the carbon-carbon double bond.

Trimerization, as used in this disclosure, is defined as the combination of any two, three, or more olefins, wherein the number of olefin, i.e., double, bonds is reduced by two. Reactants applicable for use in the trimerization process of this invention are olefinic compounds which can a) self-react, i.e., trimerize, to give useful products. E.g., the self-reaction of ethylene can give 1-hexene, and the self-reaction of 1,3-butadiene can give 1,5-cyclooctadiene; and/or b) react with other olefinic compounds, i.e., co-trimerize, to give useful products. For example, the co-trimerization of ethylene plus hexene can give 1- decene and/or 1-tetradecene, co-trimerization of ethylene and 1-butene gives one octene, co-trimerization of 1-decene and ethylene can give 1-tetradecene and/or 1-docosene, or co-trimerization of 1,3-butadiene and 1,5-hexadiene can give 1,5-cyclooctadecadiene. For example, the number of olefin bonds in the combination of three ethylene units is reduced by two, to one olefin bond, in 1-hexene. In another example, the number of olefin bonds in the combination of two 1,3-butadiene units, is reduced by two, to two olefin bonds in 1,5-cyclooctadiene. As used herein, the term "trimerization" is intended to include dimerization of diolefins, as well as "co-trimerization", both as defined above.

Suitable trimerizable olefin compounds are those compounds having from about 2 to about 30 carbon atoms per molecule and having at least one olefinic double bond. Exemplary mono-olefin compounds include, but are not limited to, acyclic and cyclic olefins such as ethylene, propylene, 1-butene, 2-butene, isobutylene, 1-pentene, 2-pentene, 1-hexene, 2-hexene, 3-hexene, 1-heptene, 2-heptene, 3-heptene, the four normal octenes, the four normal nonenes, and mixtures of any two or more thereof. Exemplary diolefin compounds include, but are not limited to, 1,3-butadiene, 1,4-pentadiene, and 1,5-hexadiene. If branched and/or cyclic olefins are used as reactants, while not wishing to be bound by theory, it is believed that steric hindrance could hinder the trimerization process. Therefore, the branched and/or cyclic portion(s) of the olefin preferably should be distant from the carbon-carbon double bond.

Catalyst systems produced in accordance with this invention preferably are employed as trimerization catalyst systems.

### Reaction Conditions

The reaction products, i.e., trimers and/or polymers, can be prepared from the catalyst systems of this invention by solution reactions, slurry reactions, and/or gas phased reaction techniques using conventional equipment and contacting processes. Contacting of the monomer or monomers with the catalyst system or with the polymerization catalyst system and trimerization/oligomerization cocatalyst system, can be effected by any manner known in the art of homogeneous (liquid) or heterogeneous (solid) catalyst systems. One convenient method is to suspend the catalyst system in an organic medium and to agitate the mixture to maintain the catalyst system in suspension throughout the trimerization, oligomerization and/or polymerization process. Other known contacting methods such as fluidized bed, gravitating bed, and fixed bed can also be employed. One convenient method with the catalyst/cocatalyst systems is to suspend a polymerization catalyst system in an organic medium and to agitate the mixture to maintain the polymerization catalyst system in suspension throughout the trimerization and/or polymerization process. An inventive cocatalyst system can then be added. A polymerization catalyst system and inventive cocatalyst system, preferably, can also be fed simultaneously to a polymerization reactor, via one or more catalyst and/or cocatalyst system feed streams. Other contacting methods such as fluidized bed, gravitating bed, and fixed bed can also be employed with all these catalyst systems.

Reaction temperatures and pressures can be any temperature and pressure which can trimerize, oligomerize, and/or polymerize the olefin reactants. Generally, reaction temperatures are within a range of about 0° to about 250°C. Preferably, reaction temperatures within a range of about 60° to about 200°C and most preferably, within a range of 80° to 150°C are employed. Generally, reaction pressures are within a range of about atmospheric to about 2500 psig. Preferably, reaction pressures within a range of about atmospheric to about 1000 psig and most preferably, within a range of 300 to 700 psig are employed.

Too low of a reaction temperature can produce too much undesirable insoluble product, and too high of a temperature can cause decomposition of the catalyst system and reaction products. Too low of a reaction pressure can result in low catalyst system activity. Too high of a pressure can cause production of too much undesirable insoluble product.

Optionally, hydrogen can be added to the reactor to accelerate the reaction and/or increase catalyst system activity.

The catalyst systems of this invention are particularly suitable for use in trimerization and/or oligomerizations. The slurry process is generally carried out in an inert diluent (medium) such as a paraffin, cycloparaffin, or aromatic hydrocarbon. Exemplary reactor diluents include, but are not limited to, isobutane and cyclohexane. Isobutane can decrease the swelling of the polymer product. However, a homogeneous trimerization/oligomerization cocatalyst system is more soluble in cyclohexane. Therefore, a preferred diluent for a homogeneous trimerization or oligomerization process is cyclohexane, and a preferred diluent for a heterogeneous trimerization or oligomerization process is isobutane. When the reactant is predominately ethylene, a temperature in the range of about 0° to about 300°C generally can be used. Preferably, when the reactant is predominately ethylene, a temperature in the range of about 60° to about 150°C is employed.

Any amount of polymerization catalyst system and cocatalyst system can be present in a polymerization reactor, in order to produce a polymer with a desired set of optimal properties, such as density, melt index, high load melt index and molecular weight. Usually, up to about 40 parts by weight of a supported, i.e., heterogeneous, cocatalyst system can be present for each part by weight of polymerization catalyst system. Preferably, about 1 to about 25 parts cocatalyst system for each part polymerization catalyst system, and most preferably, 3 to 15 parts by weight cocatalyst system for each part polymerization catalyst system are present, to produce a polymer with desirable physical and processing characteristics.

### Products

The olefinic and/or polymeric products of this invention have established utility in a wide variety of application such as, for example, as monomers for use in the preparation of homopolymers, copolymers, and/or terpolymers. The polymeric products of this invention have established utility in a wide variety of applicaton such as for example, polyethylene.

The further understanding of the present invention and its advantages will be provided by reference to the following examples.

### Examples

Various, equivalent abbreviations are used throughout the disclosure and examples. Some of these include triethylaluminum as TEA, Al(C₂H₅)₂; diethylaluminum chloride as DEAC, (Al(C₂H₅)₂Cl); chromium (III) 2-ethylhexanoate as Cr(EH)₃, CrEH, CrEH₃; hydrogen pyrrolide as pyrrole, Py, PyH, (C₄H₅N); chromium (III) acetylacetonate as Cr(acac)₃, Cracac₃, Cracac, Cr(C₅H₇O₂)₃; chromium (III) pyrrolide as CrPy₂, [Na(C₄H₁₀O₂)₂] (Cr(C₄H₄N)₃Cl(C₄H₁₀O₂)], [Na(DME)₂] [Cr(C₄H₄N)₃Cl(DME)], [Na(DME)₂] [Cr(Py)₃Cl(DME)], Product V, Compound V; chromium (III) chloride tristetrahydrofuran as CrCl₃THF₃, CrCl₃(THF)₃; 2,5-dimethylpyrrole as, hydrogen 2,5-dimethylpyrrolide, C₆H₉N, 2,5-DMP; butene as C₄=; 1-hexene as 1-C₆=; hexene as C₆=; octene as C₈=; decene as C₁₀=; dodecene as C₁₂=; tetradecene as C₁₄=.

### Example I

### Run 1001

0.14 g (0.29 mmol) of chromium (III) 2-ethylhexanoate (CrEH₃), [Cr(C₈H₁₅O₂)₃], was weighed into a 25 ml pressure tube. The tube was capped with a self sealing crown cap. 0.062 ml (0.89 mmol) pyrrole (PyH), [C₄NH₅] and cyclohexane, used as a diluent, were added via syringe to form a solution, which was about 8 ml total volume.

0.9 ml of a 1.1 M solution (0.99 mmol) of triethylaluminum (TEA), [Al(C₂H₅)₃], in heptane and a 0.9 ml aliquot of the CrEH₃/PyH solution were added under a counterflow of ethylene (CP grade) to a 1 liter autoclave reactor, containing 300 mL cyclohexane, to form a catalyst system. The reactor was sealed and ethylene addition stopped until the reactor temperature reached a reaction temperature of 80°C. The ethylene pressure was increased to a total reactor pressure of 550 psig. Ethylene was then fed on demand for a 30 minute run time. At the end of the run, a sample of the liquid reaction product mixture was taken and analyzed via capillary gas chromatography. The remaining reaction product mixture was evaporated and the amount of solid product was determined. The results are summarized below in Table XXIII.

### Run 1002

The procedure described in Run 1001 was followed except that 8 ml of a 1.1 M solution (8.8 mmol) of TEA in heptane was added directly to the CrEH₃/PyH solution to form a solution (10 ml total volume) and not to the reactor. A 0.7 ml aliquot of the CrEH₃/PyH/TEA solution was added to the autoclave reactor. No additional TEA was introduced into the reactor. The results are summarized below in Table XXIII.

### Run 1003

The procedure described in the Run 1002 was followed except that 0.10g (0.29 mmol) chromium (III) acetylacetonate (Cracac₃), [Cr(C₅H₇O₂)₃], was substituted for CrEH₃ and 6 ml of a 1.1 M solution TEA (6.6 mmol) in heptane was used in the formation of a Cracac₃/PyH/TEA solution (8 ml total volume). A 1.4 ml aliquot of the Cracac₃/PyH/TEA solution was added to the autoclave reactor. The results are summarized below in Table XXIII.

### Run 1004

The procedure described in Run 1001 was followed except that 0.9 ml of a 1 M solution (0.9 mmol) of diethylaluminum chloride (DEAC), [AlCl(C₂H₅)₂], in hexanes was added to the CrEH₃/PyH solution to form a CrEH₃/PyH/DEAC solution. A 0.65 ml aliquot of the CrEH₃/PyH/DEAC solution and 0.9 ml of a 1.1 M solution (0.99 mmol) of TEA in heptane were added to the autoclave reactor. The results are summarized below in Table XXIII.

### Run 1005

The procedure described in Run 1001 was followed except that 0.9 ml of a 1 M solution (0.9 mmol) of DEAC in hexanes was added to the CrEH₃/PyH solution and the resultant CrEH₃/PyH/DEAC solution was aged for 1 day at ambient temperature and pressure, under dry nitrogen. A 0.65 ml aliquot of the aged CrEH₃/PyH/DEAC solution + 0.9 ml of a 1.1 M solution (0.99 mmol) of TEA in heptane were added to the autoclave reactor. The results are summarized below in Table XXIII.

### Run 1006

The procedure described in Run 1001 was followed except that a solution was prepared using 0.13 ml pyrrole. Additionally, 1.0 ml of a 0.1 M solution (0.1 mmol) of DEAC in hexanes was added along with the TEA to the reactor. A 0.9 ml aliquot of the CrEH₃/PyH solution was used. The results are summarized below in Table XXIII.

### Run 1007

The procedure described in Run 1003 was followed except that 3 ml of a 1.9 M solution (5.7 mmol) of TEA in toluene was used and toluene was substituted for the cyclohexane diluent in the formation of the CrEH₃/PyH/TEA solution. Thus, an excess of toluene was present in the reactor. A 0.9 ml aliquot of the CrEH₃/PyH/TEA solution was used. The results are summarized below in Table XXIII.

### Comparative Run 1008

This run is a comparative example.

The procedure described in Run 1002 was followed except that 0.10 g of a chromium (III) pyrrolide (CrPy₃), [Cr(C₄H₄N)₃ClNa(C₄H₁₀O₂)₃] (0.17 mmol) was substituted for CrEH₃, and a solution was prepared using 0.04 ml (0.52 mmol) PyH and 3.5 ml of a 1.1 M TEA (3.85 mmol) in heptanes. The final solution volume was about 5 ml. A 1.0 ml aliquot of the CrPy₃/PyH/TEA solution was used. The results are summarized below in Table XXIII.

### Run 1009

The procedure described in Run 1008 was followed except that 1.8 ml of a 1.9 M TEA solution (3.42 mmol) in toluene was used, and toluene was substituted for cyclohexane in the formation of the CrPy₃/PyH/TEA solution. Thus, an excess of toluene was present in the reactor. A 1.4 ml aliquot of the CrPy₃/PyH/TEA solution was used. The results are summarized below in Table XXIII.

### Run 1010

The procedure described in Run 1008 was followed except that no neat PyH was added during the preparation of a CrPy₃/TEA solution. A 1.4 ml aliquot of the CrPy₃/TEA solution, in cyclohexane, was used. The results are summarized below in Table XXIII.

### Run 1011

The procedure described in Run 1009 was followed except that no neat PyH was added during the preparation of a CrPy₃/TEA solution. A 1.4 ml aliquot of the CrPy₃/TEA solution, in toluene, was used. Thus, an excess of toluene was present in the reactor. The results are summarized below in Table XXIII.

**Table XXIII**

| Homogeneous Catalyst Systems | | | | | | | | | | | | |
|---|---|---|---|---|---|---|---|---|---|---|---|---|
| Run | mg Cr (elemental) Charged | Activity (g product/ g Cr/Hr | Total Product Yield. g | Wt. % Liquid | Wt. % Solid | Liquid Product Distribution, Weight Percent | | | | | | |
| | | | | | | C₆= | 1-C₆= | C₆= | C₈= | C₁₀= | C₁₂= | C₁₄= |
| 1001 | 1.6 | 11,000 | 8.8 | 94 | 6 | 2 | - | 89^{a} | 2 | 6 | <1 | <1 |
| 1002 | 0.96 | 8,800 | 4.2 | 88 | 12 | 1 | 87 | 5 | 2 | 4 | <1 | <1 |
| 1003 | 2.6 | 9,800 | 12.8 | 64 | 36 | 2 | 81 | 4 | 4 | 5 | 1 | 1 |
| 1004 | 1.1 | 15,000 | 8.5 | 98 | 2 | <1 | - | 92^{a} | 1 | 6 | <1 | <1 |
| 1005 | 1.1 | 26,000 | 14.9 | 98 | 2 | <1 | 88 | 4 | 6 | <1 | <1 | <1 |
| 1006 | 1.5 | 12,000 | 9.0 | 98 | 2 | <1 | - | 97^{a} | 1 | 2 | <1 | <1 |
| 1007 | 2.6 | 3,500 | 4.6 | 50 | 50 | 2 | 86 | 4 | 3 | 3 | <1 | <1 |
| 1008 | 2.5 | 8,300 | 10.5 | >99 | <1 | 1 | 83 | 5 | <1 | 8 | <1 | 1 |
| 1009 | 2.5 | 4,100 | 5.2 | 99 | 1 | 1 | 88 | 4 | 1 | 6 | <1 | <1 |
| 1010 | 2.5 | 7,100 | 8.9 | 97 | 3 | <1 | 82 | 6 | 1 | 8 | <1 | 1 |
| 1011 | 2.5 | 3,300 | 4.2 | 98 | 2 | <1 | 89 | 4 | 1 | 5 | <1 | <1 |

| | | | | | | | | | | | | |
|---|---|---|---|---|---|---|---|---|---|---|---|---|
| ^{a}Total hexenes. | | | | | | | | | | | | |

### Example II

It should be noted that the results in Table XXIII, from Example X, and the results in Table XXIV, from Example XI, are not directly comparable, due to reactions conducted in different reactors, under different conditions, by the use of different ethylene and cyclohexane feedstocks, as well as different diluents. However, direct comparisons within each Example can be made.

### Run 2001

0.30 g (0.62 mmol) of chromium (III) 2-ethylhexanoate (CrEH₃) (10.15 wt% Cr) was combined with 0.12 ml (1.73 mmole) of neat pyrrole (PyH) in 10 ml of toluene. 2.8 ml of 1.9 M triethylaluminum (TEA) solution (5.32 mmol) in toluene was added, and the CrEH₃/PyH/TEA solution was stirred for 30 minutes under dry nitrogen, at ambient temperature and pressure. The dark brown CrEH₃/PyH/TEA solution was filtered and excess toluene was removed by vacuum stripping, which resulted in 1.0 ml of a dark brown oil, used as catalyst system. Under a counterflow of ethylene, 0.5 ml (0.15g CrEH₃;15.2 mg Cr) of the catalyst system and 4.0 ml of nonane (reactor internal standard) were added to a 2 liter autoclave reactor at 80°C., which contained 1.2 liters of cyclohexane. The reactor was then pressurized with ethylene to 550 psig, and the reaction was run for 30 minutes, with ethylene being fed on demand.

The results are summarized below in Table XXIV.

### Run 2002

The procedure described in Run 2001 was followed except that diethylaluminum chloride was added in addition to the CrEH₃/PyH/TEA solution.

0.53 g (1.10 mmol) of CrEH₃ (10.15 wt. % Cr) was combined with 0.52 ml (7.5 mmole) of neat PyH in 15 ml of toluene and stirred for 5 min. 9.0 ml of a 1.9 M TEA solution (17.1 mmol) in toluene was added, and the CrEH₃/PyH/TEA solution was stirred overnight under dry nitrogen at ambient temperature and pressure. Excess toluene was removed from the resultant dark brown solution via vacuum stripping, which resulted in 2.5 ml of a dark brown oil. 0.5 ml (10.8mg; 0.21 mmole Cr) of the dark brown oil was combined with 1.0 ml of a 0.87 M (0.87 mmol) diethylaluminum chloride (DEAC) solution in nonane, and the CrEH₃/PyH/TEA/DEAC solution was stirred overnight under dry nitrogen, at ambient temperature and pressure. The resultant product was used as the catalyst system. Under a counterflow of ethylene, 1.3 ml (9.4mg Cr; 0.18 mmole Cr) of the catalyst system and 4.0 ml of nonane (reactor internal standard) were charged directly to a 2 liter reactor at 80°C., which contained 1.2 liters of cyclohexane. The reactor was then pressurized with ethylene to 550 psig, and the reaction was run for 30 minutes with ethylene being fed on demand.

The results are summarized below in Table XXIV.

### Run 2003

0.33 g (0.68 mmol) of CrEH₃ (10.15 wt. % Cr) was combined with 0.13 ml (1.87 mmole) of neat PyH in 10 ml of toluene and stirred 5 min. 1.9 ml of a 1 M (1.9 mmol) DEAC solution in hexanes was added and the CrEH₃/PyH/DEAC solution was stirred for 30 minutes under dry nitrogen, at ambient temperature and pressure, which resulted in a light yellow/green solution. 5.1 ml of a 1.9 M (9.7 mmol) diethylaluminum chloride (DEAC) solution in toluene was added, and the CrEH₃/PyH/DEAC/TEA solution was stirred for 0.5 hr, which resulted in a dark yellow/brown solution. Excess toluene and hexane was removed from the dark yellow/brown CrEH₃/PyH/DEAC/TEA solution via vacuum stripping, with a dark yellow/brown oil remaining. The yellow/brown oil was dissolved and brought to a total volume of 25 ml in cyclohexane and used as a catalyst system (1.32mg Cr/ml). Under a counterflow of ethylene, 7.0ml (9.2mg Cr; 0.178 mmole Cr) of the catalyst system and 4.0 ml of nonane (reactor internal standard) were charged directly to a 2 liter reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2004

The procedure described in Run 2002 was followed, except that the CrEH₃/PyH/TEA/DEAC solution was diluted with cyclohexane prior to charging to the reactor, and dihydrogen gas (H₂) (50 psig) was added to the reactor prior to pressurizing the reactor with ethylene.

0.30 g (0.62 mmol) of CrEH₃ (10.15% Cr) was combined with 0.12 ml (1.73 mmole) of neat PyH in 10 ml of toluene. 1.7 ml of a 1 M (1.7 mmol) DEAC solution in hexanes was added and the CrEH₃/PyH/DEAC solution was stirred for 5 minutes under dry nitrogen, at ambient temperature and pressure. 1.8 ml of a 1.9 M (3.42 mmol) TEA solution in toluene was added and the CrEH₃/PyH/DEAC/TEA solution was stirred for 30 minutes under dry nitrogen, at ambient temperature and pressure. The resultant dark brown solution was filtered and excess toluene and hexanes were removed via vacuum stripping, which resulted in 0.8 ml of a dark yellow/brown oil and was used as a catalyst system. Under a counterflow of ethylene, 0.4ml (15.2mg Cr; 0.29 mmole Cr) of the catalyst system and 4.0 ml of nonane (reactor internal standard) were charged directly to the 2 liter reactor at 80°C., which contained 1.2 liters of cyclohexane. 50 psig of dihydrogen (H₂) gas was charged to the reactor, followed by pressurization with ethylene to 550 psig. The reaction was run for 30 minutes with ethylene being fed on demand.

The results are summarized below in Table XXIV.

### Run 2005

In a 500 ml Schlenk flask 1.98 g (3.4 mmol) of CrPy₃ (11.1 wt. % Cr) was combined with 40 ml of toluene and 54 ml of a 1.9 M (102.6 mmol) TEA solution in toluene. The resulting dark brown reaction mixture was stirred for 1 hour under dry nitrogen, at ambient temperature and pressure. Excess toluene was removed via vacuum stripping, which resulted in 13 ml of a dark yellow/brown oil and a small quantity of a light-colored precipitate. The dark yellow/brown oil was separated, collected by syringe from the precipitate, and used as the catalyst system. 2.0 ml of the catalyst system was diluted with 27 ml of cyclohexane and aged for 3 days under dry nitrogen, at ambient temperature and pressure before using.

Under a counterflow of ethylene, 8.0 ml (9.3mg; 0.18 mmole Cr) of the catalyst system/cyclohexane solution and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2006

The procedure described in Run 2005 was followed except that less reactants were used and less aging time was used.

In a 500 ml Schlenk flask 0.25 g (0.432 mmol) of CrPy₃ (11.1 wt. % Cr) was combined with 10 ml of toluene and 3.4 ml of a 1.9 M (6.46 mmol) TEA solution in toluene. The resulting dark brown reaction mixture was stirred for 30 minutes under dry nitrogen, at ambient temperature and pressure. Excess toluene was removed via vacuum stripping, which resulted in a dark brown oil. All of the dark brown oil was diluted to a total volume of 25 ml with cyclohexane, resulting in a solution containing 1.11 mg Cr/ml, which was used as the catalyst system.

Under a counterflow of ethylene, 8.0 ml (8.88 mg; 0.171 mmole Cr) of the catalyst system/cyclohexane solution and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2007

The procedure described in Run 2005 *was* followed except that excess toluene was present in the trimerization reactor.

In a 500 ml Schlenk flask 1.98 g (3.4 mmol) of CrPy₃ (11.1 wt. % Cr) was combined with 40 ml of toluene and 54 ml of a 1.9 M (102.6 mmol) TEA solution in toluene. The resulting dark brown reaction mixture was stirred for 1 hour under dry nitrogen, at ambient temperature and pressure. Excess toluene was removed via vacuum stripping, which resulted in 13 ml of a dark yellow/brown oil and a small quantity of a light-colored precipitate. The dark yellow/brown oil was separated, collected by syringe from the precipitate, and used as the catalyst system. 2.0 ml of the catalyst system was diluted with 27 ml of cyclohexane and aged for 3 days under dry nitrogen, at ambient temperature and pressure before using.

Under a counterflow of ethylene, 0.5 ml (8.5 mg; 0.163 mmole Cr) of the catalyst system/cyclohexane solution, 4.5 ml of toluene, and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2008

0.28 g (0.802 mmol) of Cracac₃ was combined with 0.17 ml (2.45 mmol) of neat pyrrole in 10 ml of toluene and stirred under dry nitrogen, at ambient temperature and pressure for 5 minutes. Then, 6.3 ml of a 1.9 M (12.0 mmol) TEA solution in toluene was added. The resulting dark brown reaction mixture was stirred for 30 minutes under dry nitrogen, at ambient temperature and pressure. Excess toluene was removed via vacuum stripping, which resulted in a dark yellow/brown oil. All of the dark yellow/brown oil was diluted to a volume of 25 ml with cyclohexane, resulting in a solution containing 0.0112g Cracac₃/ml, which was used as the catalyst system.

Under a counterflow of ethylene, 7.0 ml (15.2 mg; 0.293 mmole Cr) of the catalyst system/cyclohexane solution and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2009

The procedure described in Run 2008 was followed except that chromium (III) naphthenate was the chromium source.

0.33 g (0.508 mmol) of CrNapth₃ (8.0 wt. % Cr) was combined with 0.12 ml (1.73 mmol) of neat pyrrole in 10 ml of toluene and stirred under dry nitrogen at ambient temperature and pressure for 5 minutes. Then, 4.6 ml of a 1.9 M (8.74 mmol) TEA solution in toluene was added. The resulting dark brown reaction mixture was stirred for 30 minutes under dry nitrogen, at ambient temperature and pressure. Excess toluene was removed via vacuum stripping, which resulted in a dark yellow/brown oil. All of the dark yellow/brown oil was diluted to a total volume of 25 ml with cyclohexane, resulting in a solution containing 1.056 mg Cr/ml, which was used as the catalyst system.

Under a counterflow of ethylene, 7.0 ml (7.39 mg; 0.142 mmole Cr) of the catalyst system/cyclohexane solution and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2010

The procedure described in Run 2008 was followed except that the chromium (III) chloride was the chromium source.

0.41 g (1.09 mmol) of CrCl₃THF₃ was combined with 0.23 ml (3.32 mmol) of neat pyrrole in 10 ml of toluene and stirred under dry nitrogen, at ambient temperature and pressure for 5 minutes. Then 8.6 ml of a 1.9 M (16.3 mmol) TEA solution in toluene was added. The resulting dark brown reaction mixture was stirred for 30 minutes under dry nitrogen, at ambient temperature and pressure. Excess toluene was removed via vacuum stripping, which resulted in a dark yellow/brown oil. 7.5 ml of nonane was added to the dark yellow/brown oil and the resultant solution was diluted to a total volume of 25 ml with cyclohexane, resulting in a solution containing 0.0164 g CrCl₃THF₃/ml. The solution was filtered and the filtrate was used as the catalyst system.

Under a counterflow of ethylene, 5.0 ml (11.38 mg; 0.219 mmole Cr) of the catalyst system/cyclohexane/nonane solution and 2.5 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2011

The procedure described in Run 2005 was followed except that excess hexene was charged to the trimerization reactor.

In a 500 ml Schlenk flask, 1.98 g (3.4 mmol) of CrPy₃ (11.1 wt. % Cr) was combined with 40 ml of toluene and 54 ml of a 1.9 M (102.6 mmol) TEA solution in toluene. The resulting dark brown reaction mixture was stirred for 1 hour under dry nitrogen, at ambient temperature and pressure. Excess toluene was removed via vacuum stripping, which resulted in 13 ml of a dark yellow/brown oil and a small quantity of a light-colored precipitate. The dark yellow/brown oil was separated, collected by syringe from the precipitate, and used as the catalyst system. 2.0 ml of the catalyst system was diluted with 27 ml of cyclohexane and aged for 3 days under dry nitrogen, at ambient temperature and pressure before using.

Under a counterflow of ethylene, 1.0 ml (16.9 mg; 0.325 mmole Cr) of the catalyst system/cyclohexane solution, 55 ml of 1-hexene, and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

### Run 2012

The procedure described in Run 2005 was followed except that chromium (II) pyrrolide (Compound I) was the chromium source.

0.30 g (about 0.85 mmol) of Compound I (CrPy₁₀THF₄) was combined with 10 ml of toluene and 6.7 ml of a 1.9 M (12.7 mmol) TEA solution in toluene. The resulting dark brown reaction mixture was stirred for 30 minutes under dry nitrogen, at ambient temperature and pressure. Excess toluene was removed via vacuum stripping, which resulted in a dark yellow/brown oil and a small quantity of a light-colored precipitate. The dark yellow/brown oil was filtered and the filtrate was diluted to a total volume of 25 ml with cyclohexane, resulting in a solution containing 0.012 g Compound I (CrPy₁₀THF₄), which was used as the catalyst system.

Under a counterflow of ethylene, 7.0 ml of the catalyst system/cyclohexane solution and 4.0 ml of nonane (reactor internal standard) were charged directly to a 2 liter autoclave reactor at 80°C., which contained 1.2 liters of cyclohexane. The reactor was then pressurized with ethylene to 550 psig and the reaction was run for 30 minutes with ethylene being fed on demand.

The results are summarized below in Table XXIV.

**Table XXIV**

| Catalyst Systems with Solvent Removal | | | | | | | | | | | | | |
|---|---|---|---|---|---|---|---|---|---|---|---|---|---|
| Run | mg Cr (Elemental) Charged | Molar Ratio Cr:Py: TEA:DEAC | Activity (g produCt/ g cr/hr) | Total Product Yield, g | Wt. % Liquid | Wt. % Solid | Liquid Product Distribution Weight Percent | | | | | | |
| | | | | | | | C₄= | 1-C₆= | C₆= | C₈= | C₁₀= | C₁₂= | C₁₄= |
| 2001 | 15.2 | 1:3:9:0 | 4,000 | 30.5 | 74 | 26 | 9 | 53 | 2 | 9 | 9 | 5 | 4 |
| 2002 | 9.3 | 1:7:15.5:4 | 6,000 | 23.2 | 95 | 5 | <1 | 88 | 4 | 2 | 3 | <1 | <1 |
| 2003 | 9.2 | 1:3:15:3 | 8,300 | 38.5 | 88 | 12 | <1 | 86 | 3 | 2 | 5 | <1 | <1 |
| 2004 | 15.2 | 1:3:6:3 | 4,900 | 37.0 | 96 | 4 | <1 | 91 | 2 | 2 | 3 | <1 | <1 |
| 2005 | 7.5 | 1:3:30:0 | 10,600 | 40.0 | 98 | 2 | <1 | 82 | 9 | 1 | 7 | <1 | <1 |
| 2006 | 7.2 | 1:3:15:0 | 10,800 | 38.9 | 97 | 3 | <1 | 81 | 5 | 3 | 7 | <1 | 1 |
| 2007 | 6.8 | 1:3:30:0 | 1,100 | 3.2 | 94 | 6 | 4 | 84 | 6 | 1 | 4 | <1 | <1 |
| 2008 | 11.8 | 1:3:15:0 | 17,000 | 98.9 | 95 | 5 | <1 | 84 | 5 | 1 | 6 | <1 | <1 |
| 2009 | 7.4 | 1:3:15:0 | 15,700 | 57.8 | 72 | 28 | <1 | 88 | 2 | 2 | 5 | <1 | <1 |
| 2010 | 11.4 | 1:3:15:0 | 8,000 | 45.3 | 98 | 2 | <1 | 88 | 4 | 1 | 5 | <1 | <1 |
| 2011 | 13.7 | 1:30:0:0 | 4,600 | 31.3 | - | - | <1 | 76 | 11 | 1 | 10 | <1 | 1 |
| 2012 | 10.0 | 1:2:18.5:0 | 8,800 | 44.1 | 99 | 1 | <1 | 77 | 9 | 1 | 11 | <1 | 1 |

### Example III

### Run 3001

0.21g (0.601 mmol) of Cracac₃ was combined with 0.12 ml (1.73 mmol) of neat pyrrole and 15 ml of toluene. The resulting solution was stirred under dry nitrogen, at ambient temperature and pressure for 5 minutes. Then, 6.0 ml of a 1.9 M (11.4 mmol) TEA solution in toluene was added. The resulting dark brown reaction mixture was stirred for 5 minutes under dry nitrogen, at ambient temperature and pressure. Then, 2.0 g of an aluminophosphate support (0.4 P/Al molar ratio, activated at 700°C.), prepared in accordance with U.S. 4,364,855 (1982), herein incorporated by reference, was added and the resulting slurry stirred for a period of approximately 12 hours. The product was collected by filtration, rinsed at least twice with 10 ml aliquots of toluene and pentane, until no color was observed in the filtrate and vacuum dried. The dried product was used as solid supported catalyst system.

A 2.1022 g aliquot of the solid catalyst system was added under a counterflow of ethylene to a 2 liter autoclave containing 1 liter of isobutane. Prior to the catalyst charge, 0.25 ml of a 16.5 wt% TEA solution in nonane was added to the reactor, in order to neutralize any ethylene feedstock poisons that might be present. The reactor was sealed and ethylene addition was stopped until the reactor temperature reached the desired run temperature, for example 90°C. The ethylene pressure was then increased to a total reactor pressure of 550 psig. Ethylene was fed on demand for a 30 minute run time. At the end of the run, a sample of the liquid reaction product mixture was collected and analyzed via gas chromatography. The remaining reaction mixture was evaporated and the amount of solid product was determined.

The results are summarized in Table XXV.

### Run 3002

The procedure described in Run 3001 was followed except that diethylaluminum chloride was added to the Cracac₃/PyH solution along with the TEA prior to the aluminophosphate inorganic oxide addition.

0.21 g Cracac₃ (0.60 mmol) was weighed into a 30 ml screw-capped vial. 0.12 ml of PyH (1.73 mmol) and 15 ml of toluene were added, and the resulting solution was capped and stirred for 5 minutes. Then, with continued stirring, 6 ml of a 1.9 M (11.4 mmol) TEA solution in toluene was added. After the Cracac₃/PyH/TEA solution was stirred for 5 minutes, 2.4 ml of a 1 M (2.4 mmol) DEAC solution in hexanes was added and the Cracac₃/PyH/TEA/DEAC/toluene solution was stirred for 5 minutes. 2.0 g of an aluminophosphate support (0.4 P/Al molar ratio, activated at 700°C.), prepared in accordance with U.S. 4,364,855 (1982), herein incorporated by reference, was added and the resulting slurry stirred for a period of approximately 12 hours. The product was collected by filtration and rinsed with at least two 10 ml aliquots of toluene and pentane, until no color was observed in the filtrate, and vacuum dried. The dried product was used as a solid, supported catalyst system.

A 0.5048 g aliquot of the solid catalyst system was added under a counterflow of ethylene to a 2 liter autoclave containing 1 liter of isobutane. Prior to the catalyst charge, 3.0 ml of a 1.6 wt% TEA solution in nonane was added in order to neutralize any ethylene feedstock poisons that might be present. The reactor was sealed and ethylene addition stopped until the reactor temperature reached the desired run temperature, for example 90°C. The ethylene pressure was increased to a total reactor pressure of 550 psig. Ethylene was then fed on demand for a 30 minutes run time. At the end of the run, a small sample of the liquid reaction product mixture was collected and analyzed via gas chromatography. The remaining reaction mixture was evaporated and the amount of solid product determined. Ethylene consumption was determined by a calibrated flow meter.

The results are summarized below in Table XXV.

### Run 3003

The procedure described in Run 3002 was followed except that CrEH₃ was the chromium source and no aromatic solvent was used during catalyst system preparation. Also, a supported catalyst system was prepared in-situ in the reactor.

A CrEH₃/PyH solution was prepared by mixing 0.33 g (0.69 mmole) CrEH₃ with 0.26 ml (3.75 mmole) PyH in 16 ml of pentane and aging for 4 days under dry nitrogen, at ambient temperature and pressure, prior to usage. 0.49 g of an aluminophosphate support (0.9 P/Al molar ratio, activated at 700°C.), prepared in accordance with U.S. 4,364,855 (1982), herein incorporated by reference, and 2.0 ml of a 1 M (2.0 mmol) TEA solution in hexanes were charged under a counterflow of ethylene to a 2 liter autoclave reactor at ambient temperature. Then, 1 liter of cyclohexane, 2.1 ml (4.32mg; 0.083 mmole Cr) of the CrEH₃/PyH solution, and 50 psig dihydrogen gas (H₂) were charged to the reactor.

The results are summarized below in Table XXV.

**Table XXV**

| Run | Grams Catalsyt System Charged | Molar Ratio Cr:Py: TEA:DEAC | Activity (g prod/g catalyst/hr) | Total Product Yield. g | Wt. % Liquid | Wt. % Solid | Liquid Product Distribution, Weight Percent | | | | | | |
|---|---|---|---|---|---|---|---|---|---|---|---|---|---|
| | | | | | | | C₄= | 1-C₆= | C₆= | C₈= | C₁₀= | C₁₂= | C₁₄= |
| 3001 | 2.1022 | - | 210 | 223 | 82 | 18 | 1 | 76 | 7 | 2 | 11 | 1 | 2 |
| 3002 | 0.5048 | 1:3:19:4 | 340 | 87 | 99 | 1 | 1 | 82 | 7 | 1 | 8 | <1 | 1 |
| 3003 | 0.0426^{a} | 1:5.5:22.6:0 | 450 | 224 | 88 | 12 | 6 | 61 | 6 | 5 | 13 | 2 | 3 |

| | | | | | | | | | | | | | |
|---|---|---|---|---|---|---|---|---|---|---|---|---|---|
| a) Grams of only Cr(EH)₃ charged; mass of other catalyst system components are excluded. | | | | | | | | | | | | | |

### Example IV

### Run 4001

3.5 g (10 mmol) Cracac₃ was weighed into a 100 ml pressure tube. A stir bar was placed in the tube and the tube was capped with a self sealing crown cap. 40 ml toluene and 2.1 ml (30 mmol) PyH were added via syringe. 12 ml of a 2.5 M (30.0 mmol) n-butyl lithium solution in hexanes was slowly added. A precipitate was formed, collected, and washed with one 10 ml aliquot of toluene and two 10 ml aliquots of cyclohexane, until no color was observed in the wash solution. A total of 5.59 g solid was obtained. 0.5 ml of a 1.1 M (0.55 mmol) TEA solution in heptane and a slurry of 38 mg of the solid and cyclohexane were used in a reaction under the conditions described in Run 1001.

The results are summarized below in Table XXVI.

### Example 4002

The procedure described in Run 4001 was followed except that the solid catalyst component (88 mg collected) was prepared in a 25 ml pressure tube using 0.349 g (1 mmol) Cracac₃, 5 ml toluene, 0.14 ml (2 mmol) PyH, and 0.8 ml of a 2.5 M (2.0 mmol) n-butyl lithium solution in hexane.

0.5 ml of a 1.1 M (0.55 mmol) TEA solution in heptanes and a cyclohexane slurry containing 16 mg of the solid were used in a reaction under the conditions described in Run 1001.

The results are summarized below in Table XXVI.

### Run 4003

1.0 g of aluminophosphate support (0.4 P/Al molar ratio, activated 700°C.), prepared in accordance with U.S. 4,364,855 (1982), herein incorporated by reference, and an 93 mg aliquot of the solid described in Run 4001, were weighed into a 25 ml pressure tube. The tube was capped. 5 ml toluene and 3 ml of a 1.9 M (5.7 mmol) TEA solution in toluene were added to the tube via syringe. The resulting slurry was agitated for one day. The solid was isolated and washed with 10 ml aliquots of toluene and cyclohexane until no color was observed in the wash solution.

0.5 ml of a 1.1 M (0.55 mmol) TEA solution in heptanes and a cyclohexane slurry containing 80 mg of the solid were used in a reaction under the conditions described in Run 1001.

The results are summarized below in Table XXVI.

### Run 4004

0.7 g of aluminophosphate support (0.4 P/Al molar ratio, activated at 700°C.), prepared in accordance with U.S. 4,364,855 (1982), herein incorporated by reference, and an aliquot (53 mg) of the solid described in Run 4002 were weighed into a 25 ml pressure tube. The tube was capped. 3.5 ml toluene and 2 ml of a 1.9 M (3.8 mmol) TEA solution in toluene were added to the tube via syringe. The resulting slurry was agitated for one day. The solid was isolated and washed with 10 ml aliquots of toluene and cyclohexane until no color was observed in the wash solution.

0.5 ml of a 1.1 M (0.55 mmol) TEA solution in heptane and a cyclohexane slurry containing 78 mg of the solid were used in a reaction under the conditions as described in Run 1001.

The results are summarized below in Table XXVI.

**Table XXVI**

| Lithium-Alkyls in Catalyst Preparation | | | | | | | | | | | | |
|---|---|---|---|---|---|---|---|---|---|---|---|---|
| Run | Grams Catalyst Charged | Activity (g prod/g Cat/hr) | Total Product Yield. g | Wt . % Liquid | Wt. % solid | Liquid Product Distribution, Weight Percent | | | | | | |
| | | | | | | C₄= | 1-C₆= | C₆= | C₈= | C₁₀= | C₁₂= | C₁₄= |
| 4001 | 0.038 | 95 | 1.8 | 86 | 14 | 4 | 81 | 5 | 3 | 4 | <1 | <1 |
| 4002 | 0.016 | 540 | 4.3 | 82 | 18 | 3 | 83 | 5 | 3 | 4 | <1 | <1 |
| 4003 | 0.080 | 110 | 4.3 | 89 | 11 | 1 | 79 | 5 | 3 | 5 | 2 | 1 |
| 4004 | 0.078 | 310 | 12.2 | 84 | 16 | 1 | 78 | 6 | 2 | 8 | 1 | 1 |

### Example V

### Run 5001

0.17 g chromium (III) 2,2,6,6-tetramethyl-3,5-heptanedionate (Cr(III)TMHD) (0.28 mmol) was weighed into a 25 ml pressure tube. The tube was capped with a self sealing crown cap. 0.06ml of pyrrole (0.89 mmol) and 0.17 ml of neat (0.87 mmol) diisobutylaluminum chloride (DiBAlCl) were added via syringe to form a Cr(III)TMHD/DiBAlCl/Py solution, which was diluted to a total volume of about 8 ml with cyclohexane. 0.25 ml of a 1.1 M (0.28 mmol) TEA solution in heptane and 0.75 ml of the Cr(III)TMHD/DiBAlCl/Py solution were added to a glass bottle containing 100 ml of cyclohexane and 10 g of butadiene. The glass bottle was placed in a controlled temperature bath at 70°C., at ambient pressure, and agitated for a period of 16 hours. After 16 hours, a small sample of the liquid reaction product mixture was collected and analyzed via gas chromatography. The remaining liquid reaction product mixture was evaporated and the amount of solid product was determined.

The results are summarized below in Table XXVII.

### Run 5002

The procedure described in Run 5001 was followed except that no excess alkyl aluminum compound was present in the reactor and the catalyst was derived using the procedure described in Run 3001, as follows.

0.21 g (0.601 mmol) of Cracac₃ was combined with 0.12 ml (1.73 mmol) of neat pyrrole and 15 ml of toluene. The resulting solution was stirred under dry nitrogen, at ambient temperature and pressure for 5 minutes. Then, 6.0 ml of a 1.9 M (11.4 mmol) TEA solution in toluene was added. The resulting dark brown reaction mixture was stirred for 5 minutes under dry nitrogen, at ambient temperature and pressure. Then, 2.0 g of an aluminophosphate support (0.4 P/Al molar ratio, activated at 700°C.) prepared in accordance with U.S. 4,364,855 (1982), herein incorporated by reference, was added and the resulting slurry stirred for a period of approximately 12 hours. The product was collected by filtration, rinsed at least twice with 10 mL aliquots of toluene and pentane, until no color was observed in the filtrate and vacuum dried. The dried product was used as a solid, supported catalyst system.

A 0.28 g catalyst charge was used in the butadiene reaction.

The results are summarized below in Table XXVII.

**Table XXVII**

| Butadiene Reaction | | | | |
|---|---|---|---|---|
| Run | % Butadiene Conversion to Products | Distribution of Products, Weight % | | |
| | | 1,5-Cyclooctadiene | Other Liquids | Solid |
| 5001 | 97 | 91.9 | 0.3 | 7.8 |
| 5002 | 68 | 60.8 | 2.5 | 36.7 |

### Comparative Example VI

In the following Runs, all catalyst systems were prepared in a glove box, under dry nitrogen at ambient temperature and pressure. Transition metal compounds were weighed and combined with three (3) equivalents, (0.062) ml, pyrrole; 2 ml cyclohexane, as a solvent; and 6 ml of a 1.1 M solution of triethylaluminum (TEA) in heptane. The resulting product was shaken for times ranging from 5 minutes to 16 hours.

All runs were carried out in a 1 liter autoclave reactor containing 300 ml cyclohexane. 1.0 ml of the liquid catalyst systems were diluted in cyclohexane and were added to the reactor under a counterflow of ethylene (CP grade). The reactor was sealed and ethylene addition stopped until the reactor temperature reached a reaction temperature of 80°C. The ethylene pressure was increased to a total reactor pressure of 550 psig. Ethylene was fed on demand for a 30 minute run time. If necessary, heat was applied to maintain a reactor temperature of 80°C.

At the end of each run, a sample of the liquid reaction product mixture was taken and analyzed via capillary gas chromatography, on a HP-5880 gas chromatograph equipped with an FID detector and a 60 meter DB-1 column, with a 0.25 mm ID and a 0.25*µ* film. The gas chromatograph was ramped from 40°C. to 275°C. at a rate of 10°C./min, with a 20 minute hold time. Cyclohexane was used as an internal standard. The remaining reaction product mixture was evaporated and the amount of solid product produced was determined.

The results are given in Table XXVIII.

**Table XXVIII**

| Run | Metal Compound | Gram, Metal Compound | Catalyst System Appearance | Gram Catalyst Charged | Activity, (g product/ g catalyst/ hr.) | Grams, total Prod. | Weight Percent Liquid | Weight Percent Solid | Liquid Product Distribution, Weight Percent | | | | |
|---|---|---|---|---|---|---|---|---|---|---|---|---|---|
| | | | | | | | | | C₄= | 1C₆= | C₆= | C₈= | C₁₀= |
| 6001 | Ni(acac)₂ | 0.075 | dark brown solution some solids | 0.0094 | 324 | 1.52 | 95.4 | 4.6 | 4 | 65 | 4 | 7 | 9 |
| 6002 | Ni(napth)₂ | 0.285 | dark brown solution | 0.0356 | 8 | 0.14 | 29.5 | 70.5 | 74 | 0 | 0 | 4 | 0 |
| 6003 | Co(acac)₂ | 0.075 | brown solution, dark solid | 0.0094 | 59 | 0. 28 | 78.5 | 21.5 | 71 | 13 | 0 | 11 | 1 |
| 6004 | Mn(acac)₂ | 0.073 | brown-green solution | 0.0091 | 18 | 0.08 | 27.7 | 72.3 | 54 | 0 | 0 | 6 | 0 |
| 6005 | Mn(acac)₃ | 0.102 | brown solution | 0.0128 | 12 | 0.08 | 23.7 | 76.3 | 50 | 0 | 0 | 0 | 0 |
| 6006 | Cu(acac) | 0.076 | dark green solution | 0.0095 | 97 | 0.46 | 89.2 | 10.8 | 5 | 0 | 0 | 0 | 0 |
| 6007 | MoO₂(acac)₂ | 0.095 | green/ red solution some solid | 0.0119 | 16 | 0.10 | 47.7 | 52.3 | 41 | 0 | 0 | 34 | 5 |
| 6008 | TiO(acac)₂ | 0.075 | - | 0.0094 | 222 | 1.04 | 71.2 | 28.8 | 11 | 48 | 7 | 8 | 20 |
| 6009 | VO(acac)₂ | 0.077 | - | 0.0096 | 115 | 0.56 | 56.8 | 43.2 | 15 | 50 | 13 | 16 | 2 |
| 6010 | Zr(acac)₂ | 0.141 | - | 0.0176 | 29 | 0.26 | 26.8 | 73.2 | 0 | 27 | 0 | 44 | 4 |

The data in Table XXVIII show that other metal compounds can trimerize, oligomerize, and/or polymerize 1-olefins. Of these metal compounds, Ni(acac)₂, Run 6001, showed the best activity and selectivity toward trimerization.

### Example VII

In the following Example, Runs 7001-7005 demonstrate the effect of hydrolyzing the metal alkyl prior to use and the effect of the presence and absence of a pyrrole-containing compound. Runs 7006-7009 compared to Runs 7010-7013 demonstrate the effect of preparation of a catalyst system in an unsaturated hydrocarbon. Thus, Runs 7001-7006, 7009-7011, and 7013 are comparative examples.

### Runs 7001-7005

In Runs 7001-7005, the molar ratio of elemental chromium to elemental aluminum to ligand (Cr:Al:L), the catalyst components added to the reactor are 1:30:10. In Runs 7001-7003, the chromium compound was Cr(EH)₃ and the chromium compound in Runs 7004 and 7005 was Cr(Py)₃. The aluminum compound was triisobutylaluminum (Al(i-Bu)₃) and was treated in the following manner. To an approximately ten percent, by weight, solution of triisobutylaluminum in heptane was added 1.0 mole equivalent of distilled water, steadily, but in one batch, while cooling the flask containing the solution with ice water to maintain a temperature of about 10 to about 20°C. The solution was stirred vigorously during and after water addition and continued until no further gas evolution was observed. The ligand was dimethoxyethane (DME).

Runs 7001-7005 were carried out in a 2 liter autoclave reactor. The chromium compound was dissolved in 400-500 ml anhydrous n-heptane and added to the reactor, under a dry nitrogen purge. Then the appropriate volume of a stirred, treated 0.31 M solution of i-Al(Bu)₃ in heptane, as described above, was added. Then, the appropriate volume of DME was added, along with 5 ml of nonane (rector internal standard). The reactor was sealed and brought to a temperature of 80°C. in Run 7001 and 95°C. in Runs 7002-7005 and a pressure of 550 psig with ethylene. Ethylene was fed on demand for a run time of 25 minutes in Run 7001, 30 minutes in Run 7002 and 45 minutes in Runs 7003-7005.

At the end of each run, a sample of the liquid reaction product mixture was taken and analyzed via capillary gas chromatography, on a HP-5800 gas chromatograph equipped with an FID detector and a 60 meter DB-1 column, with a 0.25 mm ID and a 0.25*µ* film. The gas chromatograph was ramped from 40°C. to 275°C. at a rate of 10°C./min, with a 20 minute hold time. The remaining reaction product mixture was evaporated and the amount of solid product produced was determined.

The catalyst systems used in Runs 7006-7013 were prepared according to the following procedures. Catalyst systems in Runs 7006-7009 were prepared in the presence of toluene, an unsaturated aromatic hydrocarbon. Catalyst systems in Runs 7010-7013 were prepared in the presence of 1-hexene, an unsaturated aliphatic hydrocarbon.

### Run 7006

3.72 g of [Na(DME)₂] [CrCl(Py)₃DME] was combined with 50 ml toluene. Slowly, 26.4 ml of neat (93%) TEA was added and stirred for 30 minutes. The slurry turned dark brown. Excess solvent was removed by vacuum, resulting in a dark yellowish/brown oil and solid. About 70 ml cyclohexane was added. The resultant product was filtered and the filtrate was diluted to 200 ml with cyclohexane and 8.0 ml were charged to the reactor. The product contained 1.67 mg Cr/ml.

### Run 7007

0.35 g of chromium(III) ethylhexanoate (CrEH₃) was combined with about 15 ml of toluene, forming a deep green solution. 0.22 ml of 2,5-dimethylpyrrole (2,5-DMP) and 0.20 ml of 1-bromobutane were added. Slowly, 5.7 ml of 1.9 M TEA solution in toluene was added and stirred for 30 minutes to give a greenish, brown solution and a solid. Excess solvent was removed by vacuum and the liquid was extracted into about 15 ml cyclohexane. The resultant product was filtered and the filtrate was diluted to 25 ml with cyclohexane to form a golden colored solution, of which 7.0 ml were charged to the reactor. The product contained 0.014 g CrEH₃/ml.

### Run 7008

The procedure described in Run 7007 was followed, except that 0.22g CrEH₃ and 0.13 ml of 2,5-DMP were used. Furthermore, 0.10 ml of GeCl₄ were substituted for the 1-bromobutane. 3.4 ml of 1.9 M TEA solution in toluene was added to give a brown to brown/yellow solution and a precipitate. The final product after filtration and dilution to 25 ml with cyclohexane, was a bright gold-yellow color and contained 0.0088g CrEH₃/ml. 3.0 ml were charged to the reactor.

### Run 7009

2.070 g of CrPy₃Cl was added to 70 ml toluene and 62 ml of 1.9 M TEA solution in toluene, mixed and filtered. The filtrate volume was reduced to about 20 ml by a dynamic vacuum. The viscous brown solution was filtered again. Then, about 30 ml of pentane was added to the filtrate. After about one day, the solution was vacuum stripped of excess solvent. Then 38.1 g of alumino-phosphate (P/Al molar ratio of 0.9, activation at 700°C.), prepared in accordance with U.S. 4,364,855 were added. The slurry was stirred about 30 hours. The solid was collected by filtration and washed separately with toluene, cyclohexane and pentane. 0.4388 g of the solid catalyst system were charged to the reactor.

### Run 7010

0.21 g of [Na(DME)₂] (CrCl(Py)₃DME] was combined with about 15 ml 1-hexene. Slowly, 0.75 ml of neat (93%) TEA was added, forming a brown solution and a sticky-looking precipitate, and stirred for 30 minutes. Excess solvent was removed by vacuum. The residue was extracted into about 15 ml cyclohexane, filtered and the filtrate was diluted to 25 ml with cyclohexane. 8.0 ml (0.067 g) were charged to the reactor.

### Run 7011

The procedure described in Run 7010 was followed, except that the final catalyst system, in cyclohexane, was aged for about 24 hours prior to use. 8.0 ml (0.067 g) were charged to the reactor.

### Run 7012

0.26 g of CrEH₃ was dissolved in about 15 ml 1-hexene. 0.15 ml of 2,5-DMP and 0.13 ml of 1-bromobutane were added. Slowly, 1.0 ml of neat (93%) TEA was added and stirred for 30 minutes. Excess solvent was removed by vacuum and the liquid was extracted into about 15 ml cyclohexane. The resultant product was filtered and the filtrate was diluted to 25 ml with cyclohexane. 7.0 ml were charged to the reactor.

### Run 7013

0.21 g [Na(DME)₂] [CrCl(Py)₃DME] was combined with about 15 ml 1-hexene. Slowly, 1.0 ml of neat (93%) TEA was added, forming a dark brown solution and precipitate, and stirred for about 1 hour. The solution was decanted off and added to 1.5 g of aluminophosphate (P/Al molar ratio of 0.4, activation at 700°C.), prepared in accordance with U.S. 4,364,855, were added.

The supported catalyst system was collected by filtration, washed with 1-hexene and dried under a nitrogen purge. 0.6328 g of the solid catalyst system were charged to the reactor.

Runs 7006-7013 were carried out in a 1.2 liter autoclave reactor, containing cyclohexane. The heterogeneous, dried, supported catalyst systems (Runs 7009 and 7013) were slurried in cyclohexane to facilitate addition to the polymerization reactor, and were added to the polymerization reactor under a counterflow of ethylene (CP grade). The homogeneous, liquid, unsupported catalyst systems (Runs 7006-7008 and 7010-7012) were diluted in cylcohexane and were added to the polymerization reactor under a counterflow of ethylene (CP grade). The reactor was sealed and ethylene addition stopped until the reactor temperature reached a reaction temperature of 80°C. The ethylene pressure was increased to a total reactor pressure of 550 psig. Ethylene was then fed on demand for a 30 minute run time. At the end of the run, a sample of the liquid reaction product mixture was taken and analyzed via capillary gas chromatography, on a HP-5880 gas chromatograph equipped with an FID detector. The column was a 60 meter DB-1 column with a 0.25 mm ID and a 0.25*µ* film. The gas chromatograph was ramped from 40°C. to 275°C. at a rate of 10°C./min, with a 20 minute hold time. The remaining reaction product mixture was evaporated and the amount of solid product produced was determined.

The results of the reactions are in Table XXIX, below.

**Table XXIX**

| Run | Catalyst System | mg Cr (elemental) Charged | Activity, g product /g Cr/hr | Grams, Total Products | Weight Percent Liquid | Weight Percent Solid | Molar Ratios Cr/N/Al/L^{(a)} | Liquid Product Distribution, Weight Percent | | | | |
|---|---|---|---|---|---|---|---|---|---|---|---|---|
| | | | | | | | | C₄= | 1-C₆= | C₆= | C₈= | C₁₀= |
| 7001 | Cr(EH)_{3,} DME, i- Al(Bu)₃/ H₂O | 37.6 | 430 | 6.8 | 47 | 53 | 1/0/30/10 | 1 | 94 | <1 | 4 | <1 |
| 7002^{(b)} | Cr(EH)₃, DME, i- Al(Bu)₃/ H₂O | 37.6 | 3,100 | 59.1 | 16 | 84 | 1/0/30/10 | 1 | 94 | 1 | 1 | 2 |
| 7003 | Cr(EH)₃, DME, i- Al(Bu)₃/ H₂O | 13.2 | 4,000 | 39.7 | 64 | 36 | 1/0/30/10 | 1 | 95 | <1 | 2 | 1 |
| 7004 | Cr(Ply)₃ DME, i- Al(Bu)₃,/ H₂O | 13.5 | 830 | 8.3 | 87 | 13 | 1/0/30/10 | 1 | 83 | 6 | 2 | 7 |
| 7005 | Cr(Py)₃^{(c)} DME, i- Al(Bu)₃/ H₂O | 38.7 | 520 | 15.0 | 76 | 24 | 1/0/30/10 | 2 | 80 | 4 | 2 | 6 |
| 7006 | CrCl(PY)₃, DME, TEA | 13.5 | 7,800 | 52.6 | 99.4 | 0.6 | 1/3/30/0 | <1 | 82 | 10 | 1 | 7 |
| 7007 | Cr(EH)₃, 25 DMP, TEA, n-BuBr | 9.9 | 25,300 | 126.8 | 99.2 | 0.8 | 1/3/15/2.5 | <1 | 92 | 2 | <1 | 5 |
| 7008 | Cr(EH)₃, 25 DMP, TEA, GeCl₄ | 2.7 | 66,400 | 87.7 | 99.9 | 0.1 | 1/3/15/2.5 | <1 | 98 | < 1 | <1 | <1 |
| 7009 | CrCl(Py)₃, DME, 0.9 P/A1 | 0.397g Catalyst | 69^{d} | 14.0 | 97.1 | 2.9 | 1/3/30/0 | <1 | 87 | 7 | 1 | 3 |
| 7010 | CrCl(Py)₃, DME, TEA | 6 | 5,200 | 16.3 | 96.3 | 3.7 | 1/3/15/0 | 16 | 55 | 2 | 12 | 8 |
| 7011 | CrCl(Py)₃, DME, TEA | 6 | 5,070 | 15.9 | 96.2 | 3.8 | 1/3/15/0 | 15 | 55 | 2 | 12 | 8 |
| 7012 | Cr(EH)₃, 2,5 DMP, TEA, n-BuBr | 7.4 | 10,200 | 38.9 | 96.7 | 3.3 | 1/3/15/0 | 3 | 74 | 5 | 2 | 12 |
| 7013 | CrCl(Py)₃, DME, TEA, 0.4 P/A1 | 0.6328g Catalyst | 34^{d} | 19.0 | 56.8 | 43.2 | 1/3/15/2.5 | 9 | 45 | 3 | 10 | 10 |

| | | | | | | | | | | | | |
|---|---|---|---|---|---|---|---|---|---|---|---|---|
| ^{a)} N is nitrogen containing compound, i.e., pyrrole-containing compound; L is ligand. b) Reactor plugged with solids. ^{c)} CrCl(Py)₃ is equivalentr to Cr(Py)₂, both of which are [Na(DME)₂] [CrCl(Py)₃DME]. ^{d)} Activity is in units of g product/g·catalyst/hr. | | | | | | | | | | | | |

The data in Table XXIX shows that the presence of water (Runs 7001-7005) is detrimental to the formation of liquids, such as for example, 1-hexene. In fact, water in the reactor results in high solids formation.

Runs 7006-7013 show that catalyst systems prepared in the presence of any unsaturated hydrocarbon are effective toward trimerization. However, comparison of Runs 7006-7009, prepared in toluene, with Runs 7010-7013, prepared in 1-hexene, show that an unsaturated aromatic hydrocarbon is the preferred catalyst system preparation medium.

### Example VIII

The following Example, Runs 8001-8017, demonstrates the effect of varying the pyrrole compound, halogen, and metal additive used.

Catalyst systems used in Runs 8001-8017 were all prepared in the same general procedure. In a typical preparation, chromium(III) 2-ethylhexanoate was dissolved in toluene. Next, 3 equivalents of 2,5-dimethylpyrrole (or hydrogen pyrrolide for Runs 8014-8017) were added to the solution. The desired amount of halide additive (2 to 3 molar equivalents) was then added, followed by 15 molar equivalents of triethylaluminum (TEA). The reaction mixture was stirred for 5-10 minutes and toluene was removed under vacuum. The liquid residue was diluted to a total volume of 10 ml with cyclohexane and an aliquot was charged to the reactor as the catalyst system.

The trimerization reaction runs were carried out in a 2-liter autoclave polymerization reactor containing 1.2 liters of 85% cyclohexane as the reactor diluent. Catalyst system was charged to the reactor followed by addition of cyclohexane. The reactor temperature was brought to 80°C. at which point ethylene was introduced. The pressure was maintained at 550 psig with ethylene fed on demand. Each reaction was run for 30 minutes before shutting off ethylene. Samples were taken at the end of the run and analyzed by gas chromatography, as described in other examples.

The results of the Runs and analyses are given in Table XXX.

**Table XXX**

| Run | Additive | mg Cr Charged | Molar Ratio Cr/Py/TEA/Add | Activity,^{a} g liquid/g Cr/hour | Total Prod. Grams | Wt.% Liquid | Wt. % Solid | Liquid Product Distribution, Weight Percent | | | | |
|---|---|---|---|---|---|---|---|---|---|---|---|---|
| | | | | | | | | C₄= | 1-C₆= | C₆= | C₈= | C₁₀= |
| 8001 | None | 13.7 | 1/3/15/0 | 4700 | 33.7 | 95 | 5 | 30 | 43 | 6 | 5 | 13 |
| 8002 | DEAC | 14.2 | 1/3/15/3 | 16800 | 121.2 | 99 | 1 | <1 | 86 | 3 | 1 | 8 |
| 8003 | DEAB | 6.6 | 1/3/15/3 | 1800 | 61.5 | 98 | 2 | <1 | 93 | 2 | <1 | 4 |
| 8004 | DEA1-I | 13.7 | 1/3/15/3 | 630 | 5.0 | 86 | 14 | 4 | 72 | 8 | 2 | 12 |
| 8005 | n-BuCl | 6.8 | 1/3/15/3 | 6300 | 21.9 | 98 | 2 | 18 | 52 | 7 | 3 | 16 |
| 8006 | n-BuBr | 6.9 | 1/3/15/2.5 | 27500 | 95.0 | >99 | <1 | <1 | 91 | 2 | <1 | 5 |
| 8007 | n-BuBr | 9.9 | 1/3/15/2.5 | 25300 | 126.8 | >99 | <1 | <1 | 92 | 2 | <1 | 5 |
| 8008 | n-BuI | 6.1 | 1/3/15/3 | 2000 | 7.2 | 85 | 15 | 4 | 71 | 7 | 2 | 14 |
| 8009 | He₃SiCl | 14.6 | 1/3/15/3 | 3600 | 26.9 | 97 | 3 | 16 | 57 | 6 | 4 | 14 |
| 8010 | Me₃SiBr | 6.6 | 1/3/15/3 | 14000 | 46.6 | >99 | <1 | <1 | 86 | 4 | <1 | 8 |
| 8011 | GeCl₄ | 6.6 | 1/3/15/2 | 55000 | 181.7 | >99 | <1 | <1 | 96 | <1 | <1 | 3 |
| 8012 | GeCl₄ | 2.7 | 1/3/15/2 | 66400 | 87.7 | >99 | <1 | <1 | 98 | <1 | <1 | <1 |
| 8013 | SnCl₄ | 6.9 | 1/3/15/2 | 40600 | 140.4 | >99 | <1 | <1 | 96 | <1 | <1 | 2 |
| 8014 | DEAC | 10.8 | 1/3/15/2.5 | 6900 | 38.5 | 96 | 4 | <1 | 87 | 4 | 2 | 5 |
| 8015 | DEAB | 10.8 | 1/3/15/2.5 | 2600 | 14.3 | 97 | 3 | <1 | 89 | 3 | 2 | 4 |
| 8016 | DEAL-I | 10.8 | 1/3/15/2.5 | 70 | 0.4 | 44 | 56 | 19 | 60 | 6 3 | 4 | |
| 8017 | GeCl₄ | 10.8 | 1/3/15/2.5 | 3300 | 19.0 | 94 | 6 | <1 | 95 | 2 1 | 2 | |

| | | | | | | | | | | | | |
|---|---|---|---|---|---|---|---|---|---|---|---|---|
| ^{a)} Based on grams liquid product only. | | | | | | | | | | | | |

The data in Table XXX show that the selectivity towards 1-hexene increases in the order I<Cl<Br. The bromine-containing additives consistently have the highest selectivity for the formation of 1-hexene compared to the corresponding chloride or iodide additive. The increased production of 1-hexene also means that less byproducts (C₄=, C₈=, and C₁₀=) are being formed. The ratio of 1-hexene to internal hexenes also tends to increase in the order I<Cl<Br. Thus, the use of halides leads not only to more product, but a cleaner trimer product as well. The activity of the catalyst system increases in the order I<<Cl,Br. However, the activity between Br and Cl analogues appear to be unpredictable. For some additives (SnX₄, and AlX₃SiX) the Br is more active.

The data in Table XXX also show that the trend in selectivity to 1-hexene and activity can be extended to catalysts containing other pyrroles, as shown in Runs 8014-8017.

Overall, the best combination of activity and selectivity is obtained using GeCl₄ or SnCl₄ as the halide additives. However, it has been shown that the selectivity towards 1-hexene is also affected by the ratio of halide additive to triethylaluminum, making it possible to obtain high selectivity from other halide additives.

### Example IX

In the following Example, Runs 9001-9004 demonstrate that excess unsaturated aromatic hydrocarbon can be detrimental to trimerization and/or oligomerization. Thus, when a catalyst system is prepared in the presence of an aromatic hydrocarbon such as toluene, removal of excess aromatic hydrocarbon is preferred. The resulting liquid is then extracted or dissolved into a desired solvent such as cyclohexane or heptane. While not wishing to be bound by theory, it is believed that an aromatic hydrocarbon can compete with a monomer to be trimerized and/or oligomerized such as ethylene, for an active site of the catalyst system. Thus, it is believed that this competition can inhibit catalyst system activity.

The catalyst system used in Runs 9001-9004 was prepared using 1.35 g of chromium(III) 2-ethylhexanoate dissolved in toluene. Next, 0.86 mL (3.2 molar equivalents) of 2,5-dimethylpyrrole was added to the solution. Then 0.90 mL (3.2 molar equivalents) of n-butylbromide was added, followed by 7.60 mL (21 molar equivalents) of 93% triethylaluminum. The mixture was stirred for 5-10 minutes and toluene was removed under vacuum. The liquid residue was dissolved into 30 mL of cyclohexane, filtered, and then diluted to a total volume of 50 mL with additional cyclohexane. Four (4) mL of this solution was charged along with the desired amount of anhydrous, degassed toluene (0, 5, 10 or 15 mL) to the reactor.

The trimerization reaction runs were carried out in a 2-liter autoclave polymerization reactor containing 1.2 liters of 85% cyclohexane as the reactor diluent. Catalyst system was charged to the reactor followed by addition of cyclohexane. The reactor temperature was brought to 80°C at which point ethylene was introduced. The pressure was maintained at 550 psig with ethylene fed on demand. Each reaction was run for 30 minutes before shutting off ethylene. The total amount of ethylene consumed, i.e., fed, was measured.

The results of Runs 9001-9004 are given in Table XXXI.

**Table XXXI**

| Effect of Aromatic Hydrocarbons on Catalyst System Activity | | | |
|---|---|---|---|
| Run | Toluene Added, ml | Toluene Added, Volume %^{(a)} | Ethylene Consumed After 30 mins^{(b)}, g |
| 9001 | 0 | 0.00 | 184 |
| 9002 | 5 | 0.42 | 160 |
| 9003 | 10 | 0.83 | 127 |
| 9004 | 15 | 1.25 | 109 |

| | | | |
|---|---|---|---|
| ^{a)} Based on total value of reactor diluent. | | | |
| ^{b)} Not adjusted for solubility of ethylene in cyclohexane. | | | |

The data in Table XXXI show that the presence of an aromatic hydrocarbon, i.e., toluene, can result in a significant decrease in the activity of the catalyst system, as measured by ethylene consumption. This decrease is proportional to the amount of aromatic hydrocarbon added to the reactor.

## Claims (Claims for the following Contracting State(s): AT, BE, CH, DK, ES, FR, GR, IT, LI, LU, NL, SE)

1. A process to prepare a catalyst system comprising combining a metal source, a pyrrole-containing compound, and a metal alkyl in a mutual solvent which is a hydrocarbon selected from unsaturated hydrocarbons, and cyclohexane, thereby forgoing a preliminary reaction step between the metal source and the pyrrole-containing compound in the presence of an electron donor solvent, wherein said metal source is a chromium compound including chromium metal, wherein the pyrrole-containing compound does not comprise chromium, and wherein the metal of the metal alkyl is aluminum, lithium, magnesium, or zinc.

2. The process of claim 1, in which the mutual solvent is an unsaturated hydrocarbon.

3. The process of claim 1 or 2, wherein the unsaturated hydrocarbon is an aromatic or aliphatic hydrocarbon having less than 70 carbon atoms per molecule.

4. The process of claim 3, wherein said unsaturated hydrocarbon is an aromatic hydrocarbon having less than 20 carbon atoms per molecule.

5. The process of claim 3 or 4, wherein the aromatic hydrocarbon is toluene, benzene, xylene, mesitylene, or hexamethylbenzene.

6. The process of any of claims 2 to 5, which includes stripping excess unsaturated aromatic hydrocarbon from the resulting catalyst system.

7. The process of any one of the preceding claims, wherein said pyrrole-containing compound is pyrrole or 2,5-dimethylpyrrolide.

8. The process of any of the preceding claims, wherein said metal alkyl is a non-hydrolyzed Al, Li, Mg, or Zn alkyl.

9. The process of any of the preceding claims, wherein said metal alkyl is an aluminum alkyl.

10. The process of claim 9, wherein said aluminum alkyl is a trialkyl aluminum compound.

11. The process of claim 10, wherein said trialkyl aluminum compound is triethyl aluminum.

12. The process of any one of the preceding claims, in which a support is incorporated into the catalyst system.

13. The process of claim 12, wherein said support is an inorganic oxide such as silica, silica-alumina, alumina, fluorided alumina, silated alumina, thoria, aluminophosphate, aluminum phosphate, phosphated silica, phosphated alumina, silica-titania, coprecipitated silica/titania, fluorided/silated alumina, or a mixture thereof.

14. The process of any one of the preceding claims, which includes adding a halide source.

15. The process of claim 14, wherein the halide is a chloride or bromide, or a mixture thereof.

16. The process of claim 14 or 15, wherein said halide is provided by a compound having a formula of RₘXₙ, wherein R is an organic or inorganic radical, X is a halide, and the sum of m plus n is any number greater than 0.

17. The process of claim 16, wherein R is aluminum, silicon, germanium, hydrogen, boron, lithium, tin, gallium, indium, lead, or a mixture thereof.

18. The process of any one of the preceding claims, in which the resulting catalyst system has a relative ratio of:
(a) about 1 mole Cr of the metal source;
(b) 1 to 15 moles of the pyrrole-containing compound;
(c) 5 to 40 moles of the metal alkyl; and, if present,
(d) 1 to 30 moles of the halide.

19. The process of any one of the preceding claims, which is carried out in the absence of oxygen and water.

20. The process of any one of the preceding claims, wherein the metal source and the pyrrole-containing compound are combined prior to the addition of the metal alkyl.

21. The process of any one of the preceding claims, which comprises admixing the catalyst system prepared as a cocatalyst system with a polymerization catalyst system.

22. The process of claim 21, wherein said polymerization catalyst system comprises a chromium-, titanium-, zirconium-, and/or vanadium-containing catalyst.

23. A process to trimerize, oligomerize, or polymerize an olefin compound, the process comprising the following steps:
(a) preparing a catalyst system by combining a metal source, a pyrrole-containing compound, and a metal alkyl in a mutual solvent which is a hydrocarbon selected from unsaturated hydrocarbons, and cyclohexane, thereby forgoing a preliminary reaction step between the metal source and the pyrrole-containing compound in the presence of an electron donor solvent, wherein said metal source is a chromium compound including chromium metal, wherein the pyrrole-containing compound does not comprise chromium, and wherein the metal of the metal alkyl is aluminum, lithium, magnesium, or zinc, and
(b) carrying out a trimerization, oligomerization, or polymerization in the presence of the catalyst system obtained from step (a).

24. The process of claim 23, wherein said olefin compound has 2 to 30 carbon atoms per molecule and at least 1 olefinic double bond.

25. The process of claim 24, wherein said olefin compound is ethylene, 1-butene, 1-hexene, 1,3-butadiene, or a mixture thereof.

26. The process of any of claims 23 to 25 in which an unsaturated hydrocarbon is combined to prepare the catalyst system, but is first introduced during the trimerization, oligomerization, or polymerization process.

27. The process of claim 26, in which the unsaturated hydrocarbon used to prepare the catalyst system is an unsaturated aliphatic hydrocarbon also acting as the olefin compound which is trimerized, oligomerized, or polymerized.

## Claims (Claims for the following Contracting State(s): DE, GB)

1. A process to prepare a catalyst system comprising combining a metal source, a pyrrole-containing compound, and a metal alkyl in a mutual solvent which is a hydrocarbon selected from unsaturated hydrocarbons, and cyclohexane, thereby forgoing a preliminary reaction step between the metal source and the pyrrole-containing compound in the presence of an electron donor solvent, wherein said metal source is a chromium compound including chromium metal, wherein the pyrrole-containing compound does not comprise chromium, and wherein the metal of the metal alkyl is aluminum, lithium, magnesium, or zinc, provided that the catalyst system is not supported.

2. The process of claim 1, in which the mutual solvent is an unsaturated hydrocarbon.

3. The process of claim 1 or 2, wherein the unsaturated hydrocarbon is an aromatic or aliphatic hydrocarbon having less than 70 carbon atoms per molecule.

4. The process of claim 3, wherein said unsaturated hydrocarbon is an aromatic hydrocarbon having less than 20 carbon atoms per molecule.

5. The process of claim 3 or 4, wherein the aromatic hydrocarbon is toluene, benzene, xylene, mesitylene, or hexamethylbenzene.

6. The process of any of claims 2 to 5, which includes stripping excess unsaturated aromatic hydrocarbon from the resulting catalyst system.

7. The process of any one of the preceding claims, wherein said pyrrole-containing compound is pyrrole or 2,5-dimethylpyrrolide.

8. The process of any of the preceding claims, wherein said metal alkyl is a non-hydrolyzed Al, Li, Mg, or Zn alkyl.

9. The process of any of the preceding claims, wherein said metal alkyl is an aluminum alkyl.

10. The process of claim 9, wherein said aluminum alkyl is a trialkyl aluminum compound.

11. The process of claim 10, wherein said trialkyl aluminum compound is triethyl aluminum.

12. The process of any one of the preceding claims, which includes adding a halide source.

13. The process of claim 12, wherein the halide is a chloride or bromide, or a mixture thereof.

14. The process of claim 12 or 13, wherein said halide is provided by a compound having a formula of RₘXₙ, wherein R is an organic or inorganic radical, X is a halide, and the sum of m plus n is any number greater than 0.

15. The process of claim 14, wherein R is aluminum, silicon, germanium, hydrogen, boron, lithium, tin, gallium, indium, lead, or a mixture thereof.

16. The process of any one of the preceding claims, in which the resulting catalyst system has a relative ratio of:
(a) about 1 mole Cr of the metal source;
(b) 1 to 15 moles of the pyrrole-containing compound;
(c) 5 to 40 moles of the metal alkyl; and, if present,
(d) 1 to 30 moles of the halide.

17. The process of any one of the preceding claims, which is carried out in the absence of oxygen and water.

18. The process of any one of the preceding claims, wherein the metal source and the pyrrole-containing compound are combined prior to the addition of the metal alkyl.

19. The process of any one of the preceding claims, which comprises admixing the catalyst system prepared as a cocatalyst system with a polymerization catalyst system.

20. The process of claim 19, wherein said polymerization catalyst system comprises a chromium-, titanium-, zirconium-, and/or vanadium-containing catalyst.

21. A process to trimerize, oligomerize, or polymerize an olefin compound, the process comprising the following steps:
(a) preparing a catalyst system according to the process as defined in claim 1,
(b) carrying out a trimerization, oligomerization, or polymerization in the presence of the catalyst system obtained from step (a).

22. The process of claim 21, wherein said olefin compound has 2 to 30 carbon atoms per molecule and at least 1 olefinic double bond.

23. The process of claim 22, wherein said olefin compound is ethylene, 1-butene, 1-hexene, 1,3-butadiene, or a mixture thereof.

24. The process of any of claims 21 to 23, in which an unsaturated hydrocarbon is combined to prepare the catalyst system, but is first introduced during the trimerization, oligomerization, or polymerization process.

25. The process of claim 24, in which the unsaturated hydrocarbon used to prepare the catalyst system is an unsaturated aliphatic hydrocarbon also acting as the olefin compound which is trimerized, oligomerized, or polymerized.

## Patentansprüche (Patentansprüche für folgende(n) Vertragsstaat(en): AT, BE, CH, DK, ES, FR, GR, IT, LI, LU, NL, SE)

1. Verfahren zur Herstellung eines Katalysatorsystems, umfassend das Zusammenbringen einer Metallquelle, einer pyrrolhaltigen Verbindung und eines Metallalkyls in einem gegenseitigen Lösungsmittel, das ein Kohlenwasserstoff ist, der aus ungesättigten Kohlenwasserstoffen und Cyclohexan ausgewählt wird, unter Verzicht auf eine Reaktions-Vorstufe zwischen Metallquelle und pyrrolhaltiger Verbindung in Gegenwart eines Elektronen-spendenden Lösungsmittels, wobei die Metallquelle eine Chromverbindung, einschließlich von metallischem Chrom ist, wobei die pyrrolhaltige Verbindung kein Chrom enthält und wobei das Metall des Metallalkyls Aluminium, Lithium, Magnesium oder Zink ist.

2. Verfahren nach Anspruch 1, wobei das gegenseitige Lösungsmittel ein ungesättigter Kohlenwasserstoff ist.

3. Verfahren nach Anspruch 1 oder 2, wobei der ungesättigte Kohlenwasserstoff ein aromatischer oder aliphatischer Kohlenwasserstoff mit weniger als 70 Kohlenstoffatomen pro Molekül ist.

4. Verfahren nach Anspruch 3, wobei der ungesättigte Kohlenwasserstoff ein aromatischer Kohlenwasserstoff mit weniger als 20 Kohlenstoffatomen pro Molekül ist.

5. Verfahren nach Anspruch 3 oder 4, wobei der aromatische Kohlenwasserstoff Toluol, Benzol, Xylol, Mesitylen oder Hexamethylbenzol ist.

6. Verfahren nach einem der Ansprüche 2 bis 5, das das Strippen von überschüssigem, ungesättigtem, aromatischem Kohlenwasserstoff von dem resultierenden Katalysatorsystem umfasst.

7. Verfahren nach einem der vorhergehenden Ansprüche, wobei die pyrrolhaltige Verbindung Pyrrol oder 2,5-Dimethylpyrrolid ist.

8. Verfahren nach einem der vorhergehenden Ansprüche, wobei das Metallalkyl ein nicht hydrolysiertes Al-, Li-, Mg- oder Zn-Alkyl ist.

9. Verfahren nach einem der vorhergehenden Ansprüche, wobei das Metallalkyl ein Aluminiumaklyl ist.

10. Verfahren nach Anspruch 9, wobei das Aluminiumalkyl eine Trialkylaluminiumverbindung ist.

11. Verfahren nach Anspruch 10, wobei die Trialkylaluminiumverbindung Triethylaluminium ist.

12. Verfahren nach einem der vorhergehenden Ansprüche, wobei dem Katalysatorssystem ein Träger einverleibt wird.

13. Verfahren nach Anspruch 12, wobei der Träger ein anorganisches Oxid, wie Siliciumdioxid, Siliciumdioxid-Aluminiumoxid, Aluminiumoxid, fluoriertes Aluminiumoxid, silyliertes Aluminiumoxid, Thoriumoxid, Aluminiumphosphat, Aluminiumphosphat, phosphatiertes Siliciumdioxid, phosphatiertes Aluminiumoxid, Siliciumdioxid-Titandioxid, copräzipitiertes Siliciumdioxid/Titandioxid, fluoriertes/silyliertes Aluminiumoxid, oder ein Gemisch hiervon ist.

14. Verfahren nach einem der vorhergehenden Ansprüche, das die Zugabe einer Halogenidquelle umfasst.

15. Verfahren nach Anspruch 14, wobei das Halogenid ein Chlorid oder Bromid oder ein Gemisch hiervon ist.

16. Verfahren nach Anspruch 14 oder 15, wobei das Halogenid durch eine Verbindung der Formel RₘXₙ bereitgestellt wird, wobei R einen organischen oder anorganischen Rest bedeutet, X ein Halogenid bedeutet und die Summe von m plus n jede Zahl ist, die größer ist als 0.

17. Verfahren nach Anspruch 16, wobei R Aluminium, Silicium, Germanium, Wasserstoff, Bor, Lithium, Zinn, Gallium, Indium, Blei oder ein Gemisch hiervon ist.

18. Verfahren nach einem der vorhergehenden Ansprüche, wobei das resultierende Katalysatorsystem ein relatives Verhältnis von:
a) etwa 1 mol Cr der Metallquelle;
b) 1 bis 15 mol der pyrrolhaltigen Verbindung;
c) 5 bis 40 mol des Metallalkyls; und falls vorhanden,
d) 1 bis 30 mol des Halogenids aufweist.

19. Verfahren nach einem der vorhergehenden Ansprüche, das in Abwesenheit von Sauerstoff und Wasser durchgeführt wird.

20. Verfahren nach einem der vorhergehenden Ansprüche, wobei die Metallquelle und die pyrrolhaltige Verbindung vor der Zugabe des Metallalkyls zusammengebracht werden.

21. Verfahren nach einem der vorhergehenden Ansprüche, das das Vermischen des als Cokatalysatorsystem hergestellten Katalysatorsystems mit einem Polymerisationskatalysatorsystem umfasst.

22. Verfahren nach Anspruch 21, wobei das Polymerisationskatalysatorsystem einen Chrom-, Titan-, Zirconium- und/oder Vanadium-haltigen Katalysator enthält.

23. Verfahren zur Trimerisation, Oligomerisation oder Polymerisation einer Olefinverbindung, wobei das Verfahren die folgenden Schritte umfasst:
(a) Herstellen eines Katalysatorsystems durch Zusammenbringen einer Metallquelle, einer pyrrolhaltigen Verbindung und eines Metallalkyls in einem gegenseitigen Lösungsmittel, das ein Kohlenwasserstoff ist, ausgewählt aus ungesättigten Kohlenwasserstoffen und Cyclohexan, unter Verzicht auf eine Reaktions-Vorstufe zwischen Metallquelle und pyrrolhaltiger Verbindung in Gegenwart eines Elektronen-spendenden Lösungsmittels, wobei die Metallquelle eine Chromverbindung, einschließlich von metallischem Chrom ist, wobei die pyrrolhaltige Verbindung kein Chrom enthält und wobei das Metallalkyl Aluminium, Lithium, Magnesium oder Zink ist, und
(b) Durchführung einer Trimerisation, Oligomerisation oder Polymerisation in Gegenwart des in Schritt (a) erhaltenen Katalysatorsystems.

24. Verfahren nach Anspruch 23, wobei die Olefinverbindung 2 bis 30 Kohlenstoffatome pro Molekül und mindestens eine olefinische Doppelbindung aufweist.

25. Verfahren nach Anspruch 24, wobei die Olefinverbindung Ethylen, 1-Buten, 1-Hexen, 1,3-Butadien oder ein Gemisch hiervon ist.

26. Verfahren nach einem der Ansprüche 23 bis 25, wobei zur Herstellung des Katalysatorsystems ein ungesättigter Kohlenwasserstoff zugesetzt wird, der jedoch erst während des Trimerisations-, Oligomerisations- oder Polymerisationsverfahrens zugeführt wird.

27. Verfahren nach Anspruch 26, wobei der zur Herstellung des Katalysatorsystems verwendete ungesättigte Kohlenwasserstoff ein ungesättigter, aliphatischer Kohlenwasserstoff ist, der auch als Olefinverbindung wirkt, die trimerisiert, oligomerisiert oder polymerisiert wird.

## Patentansprüche (Patentansprüche für folgende(n) Vertragsstaat(en): DE, GB)

1. Verfahren zur Herstellung eines Katalysatorsystems, umfassend das Zusammenbringen einer Metallquelle, einer pyrrolhaltigen Verbindung und eines Metallalkyls in einem gegenseitigen Lösungsmittel, das ein Kohlenwasserstoff ist, der aus ungesättigten Kohlenwasssersoffen und Cyclohexan ausgewählt wird, unter Verzicht auf eine Reaktions-Vorstufe zwischen Metallquelle und pyrrolhaltiger Verbindung in Gegenwart eines Elektronen-spendenden Lösungsmittels, wobei die Metallquelle eine Chromverbindung, einschließlich von metallischem Chrom ist, wobei die pyrrolhaltige Verbindung kein Chrom enthält und wobei das Metall des Metallalkyls Aluminium, Lithium, Magnesium oder Zink ist, mit der Maßgabe, dass das Katalysatorsystem nicht geträgert ist.

2. Verfahren nach Anspruch 1, wobei das gegenseitige Lösungsmittel ein ungesättigter Kohlenwasserstoff ist.

3. Verfahren nach Anspruch 1 oder 2, wobei der ungesättigte Kohlenwasserstoff ein aromatischer oder aliphatischer Kohlenwasserstoff mit weniger als 70 Kohlenstoffatomen pro Molekül ist.

4. Verfahren nach Anspruch 3, wobei der ungesättigte Kohlenwasserstoff ein aromatischer Kohlenwasserstoff mit weniger als 20 Kohlenstoffatomen pro Molekül ist.

5. Verfahren nach Anspruch 3 oder 4, wobei der aromatische Kohlenwasserstoff Toluol, Benzol, Xylol, Mesitylen oder Hexamethylbenzol ist.

6. Verfahren nach einem der Ansprüche 2 bis 5, das das Strippen von überschüssigem, ungesättigtem, aromatischem Kohlenwasserstoff von dem resultierenden Katalysatorsystem umfasst.

7. Verfahren nach einem der vorhergehenden Ansprüche, wobei die pyrrolhaltige Verbindung Pyrrol oder 2,5-Dimethylpyrrolid ist.

8. Verfahren nach einem der vorhergehenden Ansprüche, wobei das Metallalkyl ein nicht hydrolysiertes Al-, Li-, Mg- oder Zn-Alkyl ist.

9. Verfahren nach einem der vorhergehenden Ansprüche, wobei das Metallalkyl ein Aluminiumalkyl ist.

10. Verfahren nach Anspruch 9, wobei das Aluminiumalkyl eine Trialkylaluminiumverbindung ist.

11. Verfahren nach Anspruch 10, wobei die Trialkylaluminiumverbindung Triethylaluminium ist.

12. Verfahren nach einem der vorhergehenden Ansprüche, das die Zugabe einer Halogenidquelle umfasst.

13. Verfahren nach Anspruch 12, wobei das Halogenid ein Chlorid oder Bromid oder ein Gemisch hiervon ist.

14. Verfahren nach Anspruch 12 oder 13, wobei das Halogenid durch eine Verbindung der Formel RₘXₙ bereitgestellt wird, wobei R einen organischen oder anorganischen Rest bedeutet, X ein Halogenid bedeutet und die Summe von m plus n jede Zahl ist, die größer ist als 0.

15. Verfahren nach Anspruch 14, wobei R Aluminium, Silicium, Germanium, Wasserstoff, Bor, Lithium, Zinn, Gallium, Indium, Blei oder ein Gemisch hiervon ist.

16. Verfahren nach einem der vorhergehenden Ansprüche, wobei das resultierende Katalysatorsystem ein relatives Verhältnis von:
a) etwa 1 mol Cr der Metallquelle;
b) 1 bis 15 mol der pyrrolhaltigen Verbindung;
c) 5 bis 40 mol des Metallalkyls; und falls vorhanden,
d) 1 bis 30 mol des Halogenids aufweist.

17. Verfahren nach einem der vorhergehenden Ansprüche, das in Abwesenheit von Sauerstoff und Wasser durchgeführt wird.

18. Verfahren nach einem der vorhergehenden Ansprüche, wobei die Metallquelle und die pyrrolhaltige Verbindung vor der Zugabe des Metallalkyls zusammengebracht werden.

19. Verfahren nach einem der vorhergehenden Ansprüche, das das Vermischen des als Cokatalysatorsystem hergestellten Katalysatorsystems mit einem Polymerisationskatalysatorsystem umfasst.

20. Verfahren nach Anspruch 19, wobei das Polymerisationskatalysatorsystem einen Chrom-, Titan-, Zirconium- und/oder Vanadium-haltigen Katalysator enthält.

21. Verfahren zur Trimerisation, Oligomerisation oder Polymerisation einer Olefinverbindung, wobei das Verfahren die folgenden Schritte umfasst:
(a) Herstellen eines Katalysatorsystems gemäß dem wie in Anspruch 1 definierten Verfahren,
(b) Durchführung einer Trimerisation, Oligomerisation oder Polymerisation in Gegenwart des in Schritt (a) erhaltenen Katalysatorsystems.

22. Verfahren nach Anspruch 21, wobei die Olefinverbindung 2 bis 30 Kohlenstoffatome pro Molekül und mindestens eine olefinische Doppelbindung aufweist.

23. Verfahren nach Anspruch 22, wobei die Olefinverbindung Ethylen, 1-Buten, 1-Hexen, 1,3-Butadien oder ein Gemisch hiervon ist.

24. Verfahren nach einem der Ansprüche 21 bis 23, wobei zur Herstellung des Katalysatorsystems ein ungesättigter Kohlenwasserstoff zugesetzt wird, der jedoch erst während des Trimerisations-, Oligomerisations- oder Polymerisationsverfahrens zugeführt wird.

25. Verfahren nach Anspruch 24, wobei der zur Herstellung des Katalysatorsystems verwendete ungesättigte Kohlenwasserstoff ein ungesättigter, aliphatischer Kohlenwasserstoff ist, der auch als Olefinverbindung wirkt, die trimerisiert, oligomerisiert oder polymerisiert wird.

## Revendications (Revendications pour l'(les) Etat(s) contractant(s) suivant(s): AT, BE, CH, DK, ES, FR, GR, IT, LI, LU, NL, SE)

1. Un procédé pour préparer un système de catalyseur comprenant la combinaison d'une source de métal, d'un composé renfermant du pyrrole et d'un alkyl-métal dans un solvant mutuel qui est un hydrocarbure choisi parmi les hydrocarbures insaturés et le cyclohexane, se passant ainsi d'une étape de réaction préliminaire entre la source de métal et le composé renfermant du pyrrole en présence d'un solvant donneur d'électrons, où ladite source de métal est un composé de chrome, y compris le métal chrome, où le composé renfermant du pyrrole ne comporte pas de chrome, et où le métal de l'alkyl-métal est l'aluminium, le lithium, le magnésium ou le zinc.

2. Le procédé de la revendication 1, où le solvant mutuel est un hydrocarbure insaturé.

3. Le procédé de la revendication 1 ou 2, où l'hydrocarbure insaturé est un hydrocarbure aromatique ou aliphatique ayant moins de 70 atomes de carbone par molécule.

4. Le procédé de la revendication 3, où ledit hydrocarbure insaturé est un hydrocarbure aromatique ayant moins de 20 atomes de carbone par molécule.

5. Le procédé de la revendication 3 ou 4, où l'hydrocarbure aromatique est le toluène, le benzène, le xylène, le mésitylène ou l'hexaméthylbenzène.

6. Le procédé de l'une des revendications 2 à 5, qui comprend l'entraînement de l'excès d'hydrocarbure aromatique insaturé du système de catalyseur résultant.

7. Le procédé de l'une des revendications précédentes, où ledit composé renfermant du pyrrole est le pyrrole ou le 2,5-diméthylpyrrolide.

8. Le procédé de l'une des revendications précédentes, où ledit alkyl-métal est un alkyle A1, Li, Mg ou Zn non hydrolysé.

9. Le procédé de l'une des revendications précédentes, où ledit alkyl-métal est un alkylaluminium.

10. Le procédé de la revendication 9, où ledit alkylaluminium est un composé de trialkylaluminium.

11. Le procédé de la revendication 10, où ledit composé de trialkylaluminium est le triéthylaluminium.

12. Le procédé de l'une des revendications précédentes, où un support est incorporé dans le système de catalyseur.

13. Le procédé de la revendication 12, où ledit support est un oxyde non organique comme la silice, la silice-alumine, l'alumine, l'alumine fluorée, l'alumine siliciée, l'oxyde de thorium, l'aluminophosphate, le phosphate d'aluminium, la silice phosphatée, l'alumine phosphatée, la silice-oxyde de titane, la silice/oxyde de titane coprécipité, l'alumine fluorée/siliciée, ou un mélange de celles-ci.

14. Le procédé de l'une des revendications précédentes, qui comprend l'addition d'une source d'halogénure.

15. Le procédé de la revendication 14, où l'halogénure est un chlorure ou un bromure, ou un mélange de ceux-ci.

16. Le procédé de la revendication 14 ou 15, où ledit halogénure est fourni par un composé répondant à la formule RₘXₙ, dans laquelle R est un radical organique ou non organique, X est un halogénure, et la somme de m plus n est n'importe quel nombre supérieur à 0.

17. Le procédé de la revendication 16, où R est l'aluminium, le silicium, le germanium, l'hydrogène, le bore, le lithium, l'étain, le gallium, l'indium, le plomb ou un mélange de ceux-ci.

18. Le procédé de l'une des revendications précédentes, où le système de catalyseur résultant a un rapport relatif de :
(a) environ 1 mole de Cr de source de métal ;
(b) 1 à 15 moles du composé renfermant du pyrrole ;
(c) 5 à 40 moles de l'alkyl-métal ; et, s'il est présent,
(d) 1 à 30 moles de l'halogénure.

19. Le procédé de l'une des revendications précédentes, qui est mis en oeuvre en l'absence d'oxygène et d'eau.

20. Le procédé de l'une des revendications précédentes, où la source de métal et le composé renfermant du pyrrole sont combinés avant l'addition de l'alkyl-métal.

21. Le procédé de l'une des revendications précédentes, qui comprend le mélange du système de catalyseur préparé comme un système de cocatalyseur avec un système de catalyseur de polymérisation.

22. Le procédé de la revendication 21, où ledit système de catalyseur de polymérisation comprend un catalyseur renfermant du chrome, du titane, du zirconium et/ou du vanadium.

23. Un procédé pour trimériser, oligomériser ou polymériser un composé d'oléfines, le procédé comprenant les étapes ci-après :
(a) la préparation d'un système de catalyseur en combinant une source de métal, un composé renfermant du pyrrole et un alkyl-métal dans un solvant mutuel qui est un hydrocarbure choisi parmi le cyclohexane, l'isobutane, l'hexane, le pentane, leurs mélanges et des hydrocarbures insaturés, se passant ainsi d'une étape de réaction préliminaire entre la source de métal et le composé renfermant du pyrrole, en présence d'un solvant donneur d'électrons, où la source de métal est un composé de chrome comprenant du métal-chrome, où le composé renfermant du pyrrole ne comporte pas de chrome et où le métal de l'alkyl-métal est l'aluminium, le lithium, le magnésium ou le zinc, et
(b) la mise en oeuvre d'une trimérisation, d'une oligomérisation ou d'une polymérisation en présence d'un système de catalyseur obtenu à partir de l'étape (a).

24. Le procédé de la revendication 23, où ledit composé d'oléfines a de 2 à 30 atomes de carbone par molécule et au moins une double liaison oléfinique.

25. Le procédé de la revendication 24, où ledit composé d'oléfines est l'éthylène, le 1-butène, le 1-hexène, le 1,3-butadiène ou un mélange de ceux-ci.

26. Le procédé de l'une des revendications 23 à 25, où un hydrocarbure insaturé est combiné pour préparer le système de catalyseur mais est tout d'abord introduit pendant le procédé de trimérisation, d'oligomérisation ou de polymérisation.

27. Le procédé de la revendication 26, où l'hydrocarbure insaturé utilisé pour préparer le système de catalyseur est un hydrocarbure aliphatique insaturé agissant également comme le composé d'oléfines qui est trimérisé, oligomérisé ou polymérisé.

## Revendications (Revendications pour l'(les) Etat(s) contractant(s) suivant(s): DE, GB)

1. Un procédé pour préparer un système de catalyseur comprenant la combinaison d'une source de métal, d'un composé renfermant du pyrrole et d'un alkyl-métal dans un solvant mutuel qui est un hydrocarbure choisi parmi les hydrocarbures insaturés et le cyclohexane, se passant ainsi d'une étape de réaction préliminaire entre la source de métal et le composé renfermant du pyrrole en présence d'un solvant donneur d'électrons, où ladite source de métal est un composé de chrome, y compris le métal chrome, où le composé renfermant du pyrrole ne comporte pas de chrome, et où le métal de l'alkyl-métal est l'aluminium, le lithium, le magnésium ou le zinc, pourvu que le système de catalyseur ne soit pas supporté.

2. Le procédé de la revendication 1, où le solvant mutuel est un hydrocarbure insaturé.

3. Le procédé de la revendication 1 ou 2, où l'hydrocarbure insaturé est un hydrocarbure aromatique ou aliphatique ayant moins de 70 atomes de carbone par molécule.

4. Le procédé de la revendication 3, où ledit hydrocarbure insaturé est un hydrocarbure aromatique ayant moins de 20 atomes de carbone par molécule.

5. Le procédé de la revendication 3 ou 4, où l'hydrocarbure aromatique est le toluène, le benzène, le xylène, le mésitylène ou l'hexaméthylbenzène.

6. Le procédé de l'une des revendications 2 à 5, qui comprend l'entraînement de l'excès d'hydrocarbure aromatique insaturé du système de catalyseur résultant.

7. Le procédé de l'une des revendications précédentes, où ledit composé renfermant du pyrrole est le pyrrole ou le 2,5-diméthylpyrrolide.

8. Le procédé de l'une des revendications précédentes, où ledit alkyl-métal est un alkyle Al, Li, Mg ou Zn non hydrolysé.

9. Le procédé de l'une des revendications précédentes, où ledit alkyl-métal est un alkylaluminium.

10. Le procédé de la revendication 9, où ledit alkylaluminium est un composé de trialkylaluminium.

11. Le procédé de la revendication 10, où ledit composé de trialkylaluminium est le triéthylaluminium.

12. Le procédé de l'une des revendications précédentes, qui comprend l'addition d'une source d'halogénure.

13. Le procédé de la revendication 12, où l'halogénure est un chlorure ou un bromure, ou un mélange de ceux-ci.

14. Le procédé de la revendication 12 ou 13, où ledit halogénure est fourni par un composé répondant à la formule RₘXₙ, dans laquelle R est un radical organique ou non organique, X est un halogénure, et la somme de m plus n est n'importe quel nombre supérieur à 0.

15. Le procédé de la revendication 14, où R est l'aluminium, le silicium, le germanium, l'hydrogène, le bore, le lithium, l'étain, le gallium, l'indium, le plomb ou un mélange de ceux-ci.

16. Le procédé de l'une des revendications précédentes, où le système de catalyseur résultant a un rapport relatif de :
(a) environ 1 mole de Cr de source de métal ;
(b) 1 à 15 moles du composé renfermant du pyrrole ;
(c) 5 à 40 moles de l'alkyl-métal ; et, s'il est présent,
(d) 1 à 30 moles de l'halogénure.

17. Le procédé de l'une des revendications précédentes, qui est mis en oeuvre en l'absence d'oxygène et d'eau.

18. Le procédé de l'une des revendications précédentes, où la source de métal et le composé renfermant du pyrrole sont combinés avant l'addition de l'alkyl-métal.

19. Le procédé de l'une des revendications précédentes, qui comprend le mélange du système de catalyseur préparé comme un système de cocatalyseur avec un système de catalyseur de polymérisation.

20. Le procédé de la revendication 19, où ledit système de catalyseur de polymérisation comprend un catalyseur renfermant du chrome, du titane, du zirconium et/ou du vanadium.

21. Un procédé pour trimériser, oligomériser ou polymériser un composé d'oléfines, le procédé comprenant les étapes ci-après :
(a) la préparation d'un système de catalyseur selon le procédé tel que défini dans la revendication 1,
(b) la mise en oeuvre d'une trimérisation, d'une oligomérisation ou d'une polymérisation en présence d'un système de catalyseur obtenu à partir de l'étape (a).

22. Le procédé de la revendication 21, où ledit composé d'oléfines a de 2 à 30 atomes de carbone par molécule et au moins une double liaison oléfinique.

23. Le procédé de la revendication 22, où ledit composé d'oléfines est l'éthylène, le 1-butène, le 1-hexène, le 1,3-butadiène ou un mélange de ceux-ci.

24. Le procédé de l'une des revendications 21 à 23, où un hydrocarbure insaturé est combiné pour préparer le système de catalyseur mais est tout d'abord introduit pendant le procédé de trimérisation, d'oligomérisation ou de polymérisation.

25. Le procédé de la revendication 24, où l'hydrocarbure insaturé utilisé pour préparer le système de catalyseur est un hydrocarbure aliphatique insaturé agissant également comme le composé d'oléfines qui est trimérisé, oligomérisé ou polymérisé.
